(19)

Europäisches
Patentamt
European
Patent Office
Office européen
des brevets

(11) **EP 4 410 274 A1**

(12) **EUROPEAN PATENT APPLICATION**
published in accordance with Art. 153(4) EPC

(43) Date of publication:
**07.08.2024 Bulletin 2024/32**

(21) Application number: **22876624.2**

(22) Date of filing: **24.06.2022**

(51) International Patent Classification (IPC):
*A61K 9/107* (2006.01)    *A61K 47/69* (2017.01)
*A61K 47/20* (2006.01)    *A61K 47/16* (2006.01)
*A61K 33/32* (2006.01)    *A61P 29/00* (2006.01)

(52) Cooperative Patent Classification (CPC):
**A61K 9/107; A61K 33/32; A61K 47/16;
A61K 47/20; A61K 47/69; A61P 29/00**

(86) International application number:
**PCT/KR2022/009070**

(87) International publication number:
**WO 2023/054849 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.09.2021 KR 20210128246**

(71) Applicant: **Industry Foundation of Chonnam
National University
Gwangju 61186 (KR)**

(72) Inventors:
• **PARK, In Kyu
Gwangju 61698 (KR)**
• **SAJI, Uthaman
Hwasun-gun, Jeollanam-do 58128 (KR)**

• **ANSUJA, Mattew
Hwasun-gun, Jeollanam-do 58128 (KR)**
• **KIM, Soo Wan
Gwangju 61704 (KR)**
• **BAE, Eun Hui
Gwangju 61701 (KR)**
• **CHOI, Hong Sang
Gwangju 61708 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

Remarks:
The complete document including Reference
Table(s) and the Sequence Listing(s) can be
downloaded from the EPO website

(54) **PHARMACEUTICAL COMPOSITION FOR TREATING INFLAMMATORY DISEASES**

(57)    The present invention relates to a pharmaceutical composition for treating inflammatory diseases, in which hydrophobic manganese oxide is loaded on micelles so as to be stably protected in vivo thereby easily being delivered to a target site. In addition, the hydrophilic and hydrophobic regions of micelles are bonded with a reactive oxygen species (ROS) cleavable linker and decomposed in response to active oxygen species at an inflammatory site, thereby efficiently releasing hydrophobic manganese oxide particles, and released hydrophobic manganese oxide can generate oxygen to change the microenvironment of the target site, thereby improving the effects of physiologically active substances loaded on the micelles.

[FIG. 4]

**Description**

[Technical Field]

The present invention relates to a pharmaceutical composition for treating inflammatory diseases.

[Background Art]

Inflammation is one of the body's defense reactions to prevent damage to living tissues by external physical stimuli, chemical stimuli such as contact with various allergens, or invasion of microorganisms such as bacteria, fungi or viruses.

Inflammation is classified into acute inflammation (immediate response, nonspecific response, days to weeks), chronic inflammation (delayed response, specific response, a few weeks or more), and subacute inflammation (intermediate stage between acute and chronic inflammations, characteristics of mixed product of polynuclear cells and monocytes).

Acute renal failure is a disease in which renal function is rapidly deteriorated in a short period of time over several hours to several days, and is a disease that causes body fluid, electrolyte or acid-base imbalance, accumulation of waste products in the body, and further death of the patient. It is common enough to be accompanied by acute renal failure in about 20% of hospitalized patients, and has diverse courses from mild renal function decline to severe disease requiring dialysis. Acute renal failure requiring dialysis has a fatality rate of up to 50%, but there is no specific treatment for acute renal failure. Although acute renal failure has various causes, it is known that tissue inflammation contributes as a common mechanism, and oxidative stress due to reactive oxygen species generated in damaged tissues has been identified as one of the main causes. Therefore, although various treatment methods for controlling oxidative stress have been attempted, these methods have not been used as therapeutics due to problems such as a short half-life and systemic side effects.

Therefore, there is a need for research into formulations that can be efficiently delivered to inflamed tissues caused by oxidative stress, effectively inhibit hypoxic environments, thereby changing the microenvironment of target tissues and maximizing drug effects.

[Summary of Invention]

[Problems to be Solved by Invention]

An object of the present invention is to provide a pharmaceutical composition for treating inflammatory diseases, in which manganese oxide particles whose surface is surface-modified with a hydrophobic substituent are supported inside micelles to maintain stability *in vivo* and to be released from inflammatory sites while circulating so as to decompose active oxygen, thereby minimizing the amplification of inflammation.

Another object of the present invention is to provide a pharmaceutical composition for treating inflammatory diseases, in which hydrophobic manganese oxide particles react with active oxygen at an inflammatory site to induce oxygen generation, thereby changing the microenvironment of a target tissue and improving effects of a bioactive substance supported on micelles.

[Means for Solving Problems]

1. A pharmaceutical composition for treating inflammatory diseases, including: manganese oxide particles having hydrophobic substituents on the surface thereof; and micelles that support the particles therein and are formed of micelle-forming molecules in which a hydrophilic portion and a hydrophobic portion are bound by a reactive oxygen species (ROS) cleavable linker.

2. The pharmaceutical composition according to the above 1, wherein the hydrophobic substituent is a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group or a halogen-containing group.

3. The pharmaceutical composition according to the above 1, wherein the manganese oxide is any one of MnO, $Mn_2O_3$, $MnO_2$, $Mn_3O_4$ and $Mn_2O_5$.

4. The pharmaceutical composition according to the above 1, wherein the manganese oxide particles have a particle diameter of 10 to 30 nm.

5. The pharmaceutical composition according to the above 1, wherein the linker is a thioketal linker (thioketal), TSPBA linker ($N^1$-(4-boronobenzyl)-$N^3$-(4-boronophenyl)-$N^1,N^1,N^3,N^3$-tetramethylpropane-1,3-diaminium) or AA linker (aminoacrylate).

6. The pharmaceutical composition according to the above 1, wherein the hydrophilic portion includes a water-soluble polymer, the hydrophobic portion includes a hydrophobic hydrocarbon group, and the reactive oxygen species (ROS) cleavable linker is a thioketal linker.

7. The pharmaceutical composition according to the above 6, wherein the water-soluble polymer is polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol.

8. The pharmaceutical composition according to the above 6, wherein the hydrophobic hydrocarbon group is a hydrocarbon group derived from hexadecylamine, heptadecylamine, stearamine, nonadecylamine, dodecylamine, N,Nbis(2-hydroxyethyl)laurylamine, linoleic acid, linolenic acid, palmitic acid, oleylamine, hexadecanoic acid, stearic acid, oleic acid, eicosenoic acid or erucic acid.

9. The pharmaceutical composition according to the above 1, wherein the manganese oxide particles are included in an amount of 5 to 10 parts by weight based on 100 parts by weight of the micelles.

10. The pharmaceutical composition according to the above 1, wherein the micelles have a particle diameter of 150 to 250 nm.

11. The pharmaceutical composition according to the above 1, wherein the inflammatory disease is renal failure, atopic dermatitis, edema, dermatitis, allergy, asthma, conjunctivitis, periodontitis, rhinitis, otitis media, sore throat, tonsillitis, pneumonia, gastric ulcer, gastritis, Crohn's disease, colitis, hemorrhoids, gout, ankylosing spondylitis, rheumatic fever lupus, fibromyalgia, psoriatic arthritis, osteoarthritis, rheumatoid arthritis, periarthritis, tendonitis, tenosynovitis, myositis, hepatitis, cystitis, nephritis, sjogren's syndrome or multiple sclerosis.

12. The pharmaceutical composition according to the above 1, further including a bioactive substance supported inside the micelles.

[Advantageous effects]

The present invention relates to a pharmaceutical composition for treating inflammatory diseases, in which hydrophobic manganese oxide is supported in micelles, such that it is stably protected *in vivo* and easily delivered to a target site.

Further, the hydrophilic and hydrophobic portions of micelles are bound by a reactive oxygen species (ROS) cleavable linker and decomposed in response to reactive oxygen species at the inflammatory site, thereby efficiently releasing hydrophobic manganese oxide particles. Further, the released hydrophobic manganese oxide may generate oxygen to change the microenvironment around the target site, thereby improving effects of a bioactive substance loaded on the micelles.

[Brief Description of Drawings]

FIGS. 1 and 2 illustrate a manufacturing process of hydrophobic manganese oxide.
FIG. 3 illustrates a manufacturing process of PTC.
FIG. 4 briefly illustrates a manufacturing process and an action mechanism of PTC-M.
FIGS. 5 to 10 show analysis results of the hydrophobic manganese oxide and polymer composite prepared by the method of Preparation Example 1.
FIGS. 11 and 12 show analysis results of the characteristics of PTC-M and PTC.
FIGS. 13 to 17 show analysis results of the changes according to $H_2O_2$ treatment in PTC, PTC-M, PC and PC-M, respectively.
FIGS. 18 to 21 show results of confirming the $H_2O_2$ removal characteristics of PTC-M.
FIG. 22 shows results of confirming the cell viability of PTC, PC, PTC-M and PC-M, respectively.
FIGS. 23 to 26 show results of confirming the uptake of PTC into cells.
FIG. 27 shows results of confirming the inflammation-reducing and apoptosisprotective effects of PTC-M under oxidative stress conditions through *in vitro* studies.
FIG. 28 shows results of confirming the biodistribution of PC-IR780 and IR780, respectively, in IRI mice.
FIG.29 shows results of confirming the biodistribution of PTC-IR780 in normal mice.
FIG. 30 shows results of quantification and comparison of PC-IR780, IR780 and PTC-IR780 fluorescence intensities in IRI mouse kidneys.
FIG. 31 shows results of confirming the biodistribution of PTC-M in IRI mice.

FIG. 32 shows results of confirming the effect of PTC-M on reducing kidney damage.

FIGS. 33 and 34 show results of confirming the effects of PTC-M on improving renal inflammation and apoptosis, respectively, in IRI mice.

[Mode for Carrying out Invention]

Hereinafter, the present invention will be described in detail.

The present invention provides a pharmaceutical composition for treating inflammatory diseases.

The composition of the present invention includes manganese oxide particles having a hydrophobic substituent on the surface (hereinafter referred to as hydrophobic manganese oxide).

Hydrophobic manganese oxide particles are treated so that a hydrophobic substituent is present on the surface of manganese oxide to allow manganese oxide to be stably supported inside the micelles.

The hydrophobic substituent may be, for example, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, halogen group, C1 to C30 ester group, or a halogen-containing group, etc.

The hydrophobic substituent may be substituted with, for example, a halogen atom, nitro, hydroxy, cyano, amino, thiol, carboxyl, amide, nitrile, sulfide, disulfide, sulfenyl, formyl, formyloxy, formylamino, formylamino or aryl.

In one embodiment of the present invention, manganese oxide may be treated with, for example, 1-dodecanethiol, 1-octadecanethiol or 1-hexanediol thus to have a hydrophobic substituent on the surface.

Manganese oxide is not limited to, but may be any one of MnO, $Mn_2O_3$, $MnO_2$, $Mn_3O_4$ and $Mn_2O_5$, preferably $Mn_3O_4$.

In one embodiment of the present invention, manganese oxide may be $Mn_3O_4$, which is synthesized by reducing manganese chloride with an alkaline solution (e.g., NaOH) to produce $Mn(OH)_2$, decomposing it into MnO, followed by being oxidized by oxygen in the air.

As described above, the surface of the manganese oxide particles may be modified with a hydrophobic substituent to be stably supported inside the micelles. The manganese oxide particles may be spread throughout the hydrophobic portion inside the micelles, and may be supported on the core. It is more preferable that manganese oxide is supported on the core to be released from the inflammatory site while stably circulating in the body.

The hydrophobic manganese oxide particles may have a particle diameter of 10 to 30 nm. When the hydrophobic manganese oxide is in the particle range, it may be evenly distributed and stably supported inside the micelles, and the reactivity with active oxygen at the target site may be increased to increase oxygen generation.

The present invention may include micelles formed of micelle-forming molecules, in which a hydrophilic portion and a hydrophobic portion are linked by a reactive oxygen species (ROS) cleavable linker.

The micelle-forming molecule may be formed by combining a hydrophilic portion and a hydrophobic portion by a reactive oxygen species (ROS) cleavable linker, and may form micelles having hydrophilicity on the outside and hydrophobicity on the inside.

The micelle-forming molecule may have a hydrophilic portion containing a water-soluble polymer and a hydrophobic portion containing a hydrophobic hydrocarbon group.

The linker is a linking group connecting a hydrophilic portion and a hydrophobic portion, and is highly sensitive to active oxygen, and reacts with the active oxygen to break the bond.

The linker may be, for example, a thioketal linker (thioketal), a TSPBA linker ($N^1$-(4-boronobenzyl)-$N^3$-(4-boronophenyl)-$N^1$,$N^1$,$N^3$,$N^3$-tetramethylpropane-1,3-diaminium) or AA linker (aminoacrylate), etc., but it is not limited thereto.

The micelle-forming molecule may be an amphipathic polymer compound in which a water-soluble polymer and a hydrophobic hydrocarbon group are covalently bonded with a thioketal linker (TL). Specifically, one end of the water-soluble polymer and one end of the hydrophobic hydrocarbon group may be covalently bonded with a thioketal linker through an amide group, respectively, and then self-assembled in an aqueous phase, thereby producing the micelles.

The thioketal linker is a linking group for connecting a water-soluble polymer and a hydrophobic hydrocarbon group, which has very high sensitivity to active oxygen and excellent bonding stability under normal physiological conditions, thus to prevent the micelles from early leaking hydrophobic manganese oxide particles during circulation in the blood.

The water-soluble polymer may have a weight average molecular weight in the range of 500 to 10,000 g/mol, preferably 800 to 5,000 g/mol, and more preferably 1,000 to 3,000 g/mol. The water-soluble polymer may be polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol, or derivatives thereof, etc., but it is not limited thereto.

The water-soluble polypeptide may be specifically selected from polyglutamic acid, polyaspartic acid, polyaspartamide, and copolymers thereof, but it is not limited thereto.

The water-soluble polysaccharides may be specifically selected from hyaluronic acid, alginic acid, dextran, pectin, chondroitin sulfate, heparin, hydroxypropyl cellulose, hydroxyethyl cellulose, methylcellulose, sodium carboxymethyl cellulose, hydroxypropyl methylcellulose acetate succinate and derivatives thereof, but it is not limited thereto.

The hydrophobic hydrocarbon group is a hydrocarbon group derived from a hydrophobic hydrocarbon compound, and may have an aliphatic hydrocarbon group having 12 to 22 carbon atoms, wherein the hydrocarbon group may have an aliphatic hydrocarbon group, specifically a straight-chain aliphatic hydrocarbon group.

The hydrophobic hydrocarbon compound may include hexadecylamine, heptadecylamine, stearamine, nonadecylamine, dodecylamine, N,N-bis(2-hydroxyethyl)laurylamine), linoleic acid, linolenic acid, palmitic acid, oleylamine, hexadecanoic acid, stearic acid, oleic acid, eicosenoic acid, or erucic acid, etc., but it is not limited thereto.

The hydrophobic hydrocarbon compound may have a weight average molecular weight of 100 to 500 g/mol, preferably 150 to 350 g/mol, and more preferably 180 to 300 g/mol, but it is not limited thereto.

As the hydrophobic hydrocarbon group forms a hydrophobic core, such that a hydrophobic core portion may have a certain degree of fluidity. The fluidity has an advantage over the hydrophobic core portion formed by a hydrophobic polymer having low fluidity especially in an aspect that it can rapidly release hydrophobic manganese oxide placed in the hydrophobic core in response to active oxygen. A long-chain aliphatic hydrocarbon group forms a hydrophobic core to impart sufficient hydrophobicity to the hydrophobic core, thereby stably supporting hydrophobic manganese oxide while providing a certain degree of fluidity, such that a thioketal linker with ROS-labile property becomes sensitive to and reacts with the active oxygen around target tissues, and thus, can be easily decomposed.

Further, compared to the case where a large hydrophobic polymer is used, even if the micelles formed by self-assembly in an aqueous phase has a relatively small size, a sufficient amount of hydrophobic manganese oxide may be stably collected in the hydrophobic core due to a certain degree of fluidity of the aliphatic hydrocarbon group.

A ratio of the weight average molecular weights between the water-soluble polymer and the hydrophobic hydrocarbon compound may satisfy Equation 1 below.

$$[\text{Equation 1}]$$

$$0.01 < \text{HC/WSP} < 0.3$$

(In Equation 1, WSP means the weight average molecular weight of a water-soluble polymer, and HC means the molecular weight of a hydrophobic hydrocarbon compound).

In Equation 1, a value of HC/WSP may be 0.01 to 0.3, specifically 0.04 to 0.25, and more specifically 0.08 to 0.2. Unlike conventional micelles, the micelles formed of water-soluble polymers and hydrophobic hydrocarbon groups, which satisfy Equation 1 above, have a smaller molecular weight of hydrophobic hydrocarbon groups and thus exhibit excellent self-assembly in the aqueous phase including a hydrophobic material, and are thermodynamically controlled rather than kinetically, thereby attaining an advantage of excellent reproducibility. Moreover, the resulting micelles have a small molecular weight in a hydrophobic region, such that these micelles preferably do not aggregate into secondary particles, have better dispersibility and stability, have a small particle diameter, and may exhibit significantly improved colloidal stability.

The composition of the present invention may include 5 to 10 parts by weight ("wt. parts") of manganese oxide particles based on 100 wt. parts of the micelles. When the hydrophobic manganese oxide is included in the micelles within the above range, the micelles may stably capture the hydrophobic manganese oxide, and the hydrophobic manganese oxide may be effectively released in the inflammation site. Further, the hydrophobic manganese oxide is included in a desirable amount to increase the amount of oxygen generated by the hydrophobic manganese oxide released in the target site, thereby changing the microenvironment of the target site to improve therapeutic effects of the bioactive substance.

In the present invention, the micelles may have a diameter of 150 to 250 nm. When the micelles have a diameter within the above range, circulation *in vivo* is facilitated, and decomposition of a thioketal link by active oxygen and release of hydrophobic manganese oxide may be performed well.

Inflammatory disease refers to any disease mediated by inflammation. For example, it may include renal failure, atopic dermatitis, edema, dermatitis, allergy, asthma, conjunctivitis, periodontitis, rhinitis, otitis media, sore throat, tonsillitis, pneumonia, gastric ulcer, gastritis, Crohn's disease, colitis, hemorrhoids, gout, ankylosing spondylitis, rheumatic fever lupus, fibromyalgia, psoriatic arthritis, osteoarthritis, rheumatoid arthritis, periarthritis, tendonitis, tenosynovitis, myositis, hepatitis, cystitis, nephritis, sjogren's syndrome or multiple sclerosis, etc., but it is not limited thereto.

The present invention provides a pharmaceutical composition for treating inflammatory diseases, further comprising a bioactive substance supported inside the micelles.

The composition of the present invention may be a composition in which various bioactive substances are supported inside the micelles. The bioactive substance may be released together with hydrophobic manganese oxide at a hypoxic site of the target site, and may exhibit higher efficacy due to the tissue microenvironment changed by oxygen generation induced from the hydrophobic manganese oxide.

Bioactive substances are physiologically active substances/biological function modifiers that can be supported inside the micelles and delivered to an individual to exhibit activity, and may include at least one selected from the group consisting of low molecular-weight drugs, gene drugs, protein drugs, extracts, nucleic acids, nucleotides, proteins, peptides, antibodies, antigens, RNA, DNA, PNA, aptamers, chemicals, enzymes, amino acids, sugars, lipids, compounds (natural and/or synthetic compounds), and elements constituting the same. Further, it may include at least one selected from the group consisting of, for example, doxorubicin, irinotecan, sorafenib, adriamycin, daunomycin, mitomycin, cisplatin, epirubicin, methotrexate, 5-fluorouracil, aclacinomycin, nitrogen mustard, cyclophosphamide, bleomycin, daunorubicin, vincristine, vinblastine, vindesine, tamoxifen, valrubicin, pirarubicin, mitoxantrone, gemcitabine, idarubicin, temozolomide, paclitaxel, dexamethasone, aldesleukin, avelumab, bevacijumab, carboplatin, regorafenib, docetaxel, doxil, gefitinib, imatinib mesylate, herceptin, imatinib, aldesleukin, keytruda, opdivo, mitomycin C, nivolumab, olaparib, pembrolizumab, rituximab, sunitinib, atezolizumab, lapatinib, and ipilimumab, but it is not limited thereto.

The bioactive substance may be a therapeutically active agent capable of providing, directly or indirectly, therapeutic, physiological and/or pharmacological effects to a human or animal organism.

The therapeutically active agent may be, for example, a general medication, a drug, a prodrug or a target group, or a drug or prodrug including the target group.

For example, the therapeutically active agent may include: anti-inflammatory agents, particularly non-steroidal anti-inflammatory drugs (NSAIDs), more particularly COX-2 inhibitors; steroidal anti-inflammatory agents; prophylactic anti-inflammatory; allergic rhinitis medicine; anti-arthritic agents; gout medication; antihemorrhoidal agents; gastric ulcer and inflammation treatment agents such as proton pump inhibitors; cardiovascular drugs, especially antihypertensives (e.g., calcium channel blockers, or calcium antagonists) and antiarrhythmics; congestive heart failure medication; muscle contraction; vasodilators; ACE inhibitors; diuretic; deoxygenation dehydrogenase inhibitors; cardiac glycosides; phosphodiesterase inhibitors; blockers; β-blockers; sodium channel blockers; potassium channel blockers; β-adrenergic agonists; platelet inhibitors; angiotensin II antagonists; anticoagulants; thrombolytic agents; bleeding medication; anemia treatment; thrombin inhibitor; antiparasitic agents; antibacterial agent; antiglaucoma agents; mast cell stabilizers; mydriatics; drugs affecting the respiratory system; alpha-adrenergic antagonists; corticosteroids; chronic obstructive pulmonary disease medication; xanthine-oxidase inhibitors; autopotency drugs and autopotency drug antagonists; antituberculous agents; antifungal agents; antiprotozoal agents; helminthic; antiviral agents, in particular respiratory antiviral agents, antiviral agents against herpes, cytomegalovirus, human immunodeficiency virus and hepatitis infections; therapeutics of leukemia and Kaposi's sarcoma; pain management agents, especially opioids, including anesthetics and analgesics, opioid receptor agonists, opioid receptor partial agonists, opioid antagonists, opioid receptor mixed agonist-antagonists; neuroleptics; sympathomimetics; adrenergic antagonists; drugs that affect neurotransmitter uptake and release; anticholinergics; therapeutics for prevention or treatment of radiation or chemotherapy effects; adipogenic agents; fat reducing agents; anti-obesity agents such as lipase inhibitors; sympathomimetics; prostaglandins; VEGF inhibitors; antihyperlipidemic agents, especially statins; drugs affecting the central nervous system (CNS) such as antipsychotic, antiepileptic and antiseizure drugs (anticonvulsants), psychoactive agents, stimulants, anxiolytics and hypnotics; antidepressants; antiparkinsonian agents; hormones and fragments thereof, such as sexual hormones; growth hormone antagonists; gonadotropin releasing hormone and its analogues; steroid hormone and its antagonists; selective estrogen modulators; growth factors; antidiabetic agents such as insulin, insulin fragments, insulin analogues, glucagon-like peptides and hypoglycemic agents; H1, H2, H3 and H4 antihistamines; peptide, protein, polypeptide, nucleic acid and oligonucleotide drugs; analogs, fragments and variants of natural proteins, polypeptides, oligonucleotides and nucleic acids, etc.; medications used to treat migraines; asthma medication; cholinergic antagonists; glucocorticoids; androgens; antiandrogen; inhibitors of adrenocorticoid biosynthesis; osteoporosis agents such as biphosphonates; antithyroid drugs; sunscreens, anti-ultraviolet protectors and filters; cytokine antagonists; antitumor agents; antialzheimer's; HMGCoA reductase inhibitors; fibrates; cholesterol absorption inhibitors; HDL cholesterol raising agents; triglyceride reducing agents; anti-aging or anti-wrinkle agents; precursor molecules for production of hormones; proteins such as collagen and elastin, antibacterial agents; anti-acne agents; antioxidants; hair treatment and skin lightening agents; sun creams, anti-ultraviolet protectors and filters; variants of human apolipoprotein; precursor molecules for production of hormones; their proteins and peptides; amino acid; plant extracts such as grape seed extract; DHEA; isoflavones; vitamins, nutrients including phytosterols and iridoid glycosides, sesquiterpene lactones, terpenes, phenolic glycosides, triterpenes, hydroquinone derivatives and phenylalkanones; antioxidants such as retinol and other retinoids including retinoic acid and coenzyme Q10; omega-3-fatty acids; glucosamine; nucleic acids, oligonucleotides, antisense drugs; enzyme; coenzyme; cytokine analogs; cytokine agonists; cytokine antagonists; immunoglobulins; antibodies; antibody medicine; gene therapy agents; lipoprotein; erythropoietin; vaccine; small molecule therapeutics for treatment or prevention of human and animal diseases such asallergies/asthma, arthritis, cancer, diabetes, growth disorders, cardiovascular disease, inflammation, immune disorders, baldness, pain, eye diseases, epilepsy, gynecological disorders, CNS diseases, viral infections, bacterial infections, parasitic infections, GI disorders, obesity and blood diseases, and the like, but they are not limited thereto.

The pharmaceutically acceptable carrier included in the pharmaceutical composition of the present invention is generally used in producing a formulation, and may include lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methylhydroxy benzoate, propylhydroxy benzoate, talc, magnesium stearate and mineral oil, etc., but it is not limited thereto. Other than the above components, the pharmaceutical composition of the present invention may further include lubricants, wetting agents, sweeteners, flavors, emulsifiers, suspending agents, preservatives and the like. Pharmaceutically acceptable carriers and formulations suitable in the art are described in detail in Remington's Pharmaceutical Sciences (19th ed., 1995). Suitable dosages of the pharmaceutical composition vary by factors such as a formulation method, administration manner, age, body weight, sex of a patient, severity of disease symptoms, food, administration time, administration route, excretion rate, response sensitization, or the like, and the dosage effective for desired treatment may be easily determined and prescribed by commonly skilled specialists in consideration of the above factors. Meanwhile, the dosage of the pharmaceutical composition of the present invention may be 0.01-2000 mg/kg (body weight) per day, but it is not limited thereto.

The pharmaceutical composition of the present invention may be administered orally or parenterally. For parenteral administration, intravenous injection, subcutaneous injection, intramuscular injection, intraperitoneal injection, transdermal administration, etc. may be used. It is preferable that the administration route of the pharmaceutical composition of the present invention is determined according to the type of disease to which it is applied. The pharmaceutical composition of the present invention may be formulated in a unit dose form using any pharmaceutically acceptable carrier and/or excipient, or may be formulated by introducing the same into a multi-dose container according to any method easily implemented by those skilled in the art, to which the present invention pertains. At this time, the formulation may have the form of a solution, suspension or emulsion in oil or aqueous medium, or the form of extract, powder, granules, tablets or capsules, and may further contain a dispersing agent or stabilizer.

Hereinafter, the present invention will be described in detail with reference to examples.

### Preparation Example

### Preparation Example 1. Preparation of hydrophobic manganese oxide

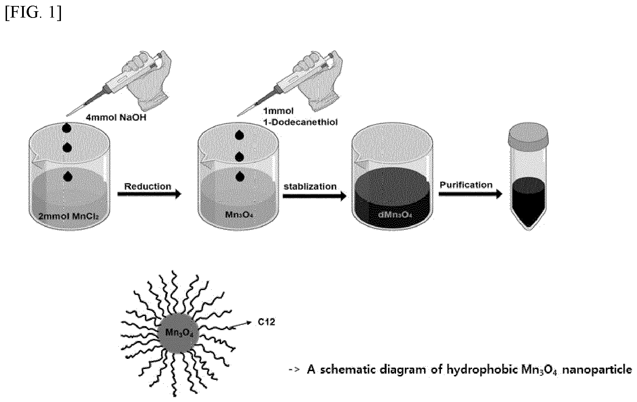

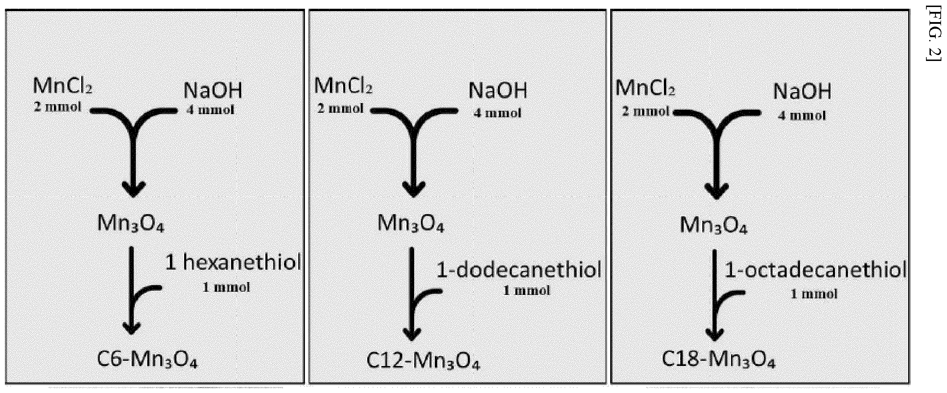

To prepare hydrophobic manganese oxide particles having a C6, C12 or C18 alkyl group, the materials in Table 1 were used, and a schematic view of hydrophobic manganese oxide particles and the hydrophobic manganese oxide production process according to a length of alkyl group are shown in FIGS. 1 and 2.

[TABLE 1]

| C6 alkyl group | C12 alkyl group | C18 alkyl group |
|---|---|---|
| Manganese (II) chloride dihydrate ($MnCl_2.2H_2O$) 2 mmol (323.74 mg in 2.5 distilled water) | | |
| NaOH4 mmol (160 mg in 2.5 ml distilled water) | | |
| 1-hexanethiol | 1-dodecanethiol | 1-octadecanethiol |
| 1 mmol (142 μl or 118.17 mg) | 1 mmol (239 μl or 202.40 mg) | 1 mmol (286.56 mg in 1ml chloroform) |

After completely dissolving $MnCl_2$, 4 mmol of NaOH was added to 2 mmol of $MnCl_2$ and stirred strongly to obtain $Mn(OH)_2$ through a reductive reaction, then it was confirmed that a sample turned brown. Then, $Mn(OH)_2$ was decomposed into MnO, and MnO was oxidized to $Mn_3O_4$ by oxygen in the air (Reduction step).

While stirring strongly 4 mmol of $Mn_3O_4$ obtained above, it reacted with 1 mmol of 1-hexanethiol, 1-dodecanethiol or 1-octadecanethiol to produce hydrophobic manganese oxide particles ($dMn_3O_4$) having each C6, C12 or C18 alkyl group on the surface of $Mn_3O_4$ (Stabilization step). The formed precipitate was washed with ethanol (5,000 rpm, 5 minutes, 5 times), vacuum dried or air dried, and the dried precipitate was washed twice with distilled water and then lyophilized to finally obtain hydrophobic manganese oxide particles (Purification step).

### Preparation Example 2. Preparation of micelles

Step 1: Preparation of thioketal linker (TL)

49 mmol of 3-mercaptopropionic acid was dissolved in 98 mmol of anhydrous acetone, and the resulting product was cooled while stirring constantly at 23°C for 6 hours until crystallization. The crystallized product was filtered, rinsed with n-hexane, and then rinsed once more with cold distilled water, followed by lyophilization.

Step 2: Synthesis of thioketal linker-conjugated polyethylene glycol (PEG-TL)

254 mg of the thioketal linker (TL) prepared in Step 1 and 200 mg of methoxypolyethylene glycol amine (PEG-AM) having a molecular weight of 2 kDa were mixed with 10 ml of N,N-dimethylformamide (DMF) and mixed at room temperature to prepare a mixed solution. Thereafter, 278 mg of 1-ethyl-3-(3-dimethylaminopropyl)carbodiimide [EDC] and 345 mg of N-hydroxysuccinimide (NHS) were added to the mixed solution prepared above, and the mixture was stirred for 16 hours. After stirring, the mixed solution was dialyzed under a condition that a molecular cut-off (MWCO) against distilled water is 1,000, so as to remove reaction byproducts, followed by lyophilization. The lyophilized powder was re-dissolved in 1 ml of DMF and precipitated in cold diethyl ether 5 times. Then, the resulting product was vacuum dried to prepare thioketal linker-conjugated polyethylene glycol (PEG-TL).

Step 3: Production of polyethylene glycol-TL-C18 (PTC)

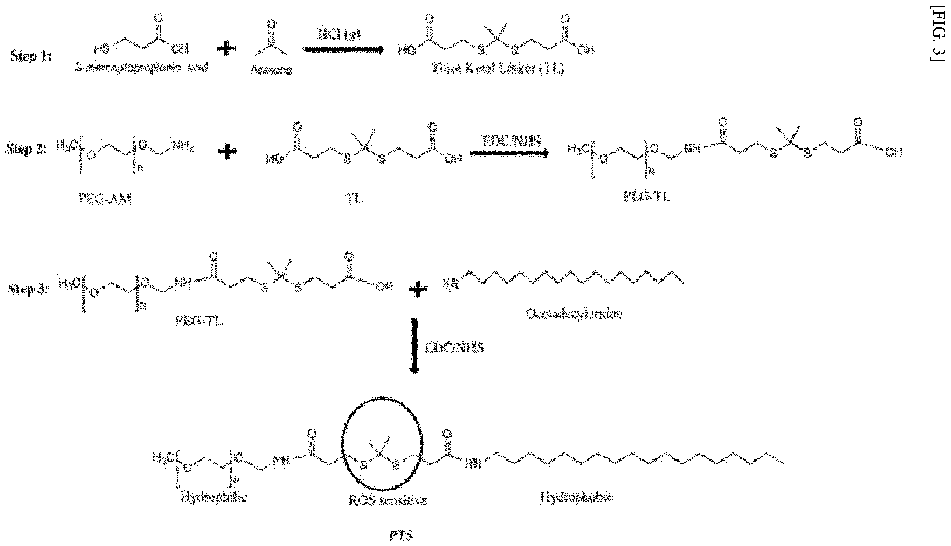

100 mg of PEG-TL prepared in Step 2 above, 80 ml of triethylamine (TEA) and 80 mg of stearamine (octadecylamine, C18) were added to 10 ml of N,N-dimethylformamide (DMF) and mixed at 80 °C. To a thoroughly mixed solution, 240 mg of 1-ethyl-3-(3-dimethylaminopropyl)carbodiimide [EDC] and 145 mg of N-hydroxysuccinimide (NHS) were added and stirred for one day. After the reaction, the solution was purified by dialysis for 2 days under a condition that a molecular cut-off (MWCO) against distilled water is 12-14 kDa, followed by lyophilization to prepare polyethylene glycol-TL-C18 (PTC) which has a hydrophilic portion and a hydrophobic portion at both ends and a ROS sensitive portion in the middle (FIG. 3).

In order to measure a critical micelle concentration (CMC) of the polymeric micelles prepared from PTC, aqueous solutions containing the polymeric micelles at various concentrations were prepared. As a fluorescent probe, $6 \times 10^{-7}$ M of pyrene was used, the excitation wavelength was set to 336 nm in a fluorescence spectrophotometer, and the emission wavelength was observed in the range of 360 to 450 nm. As a result of the measurement, the CMC of the PTC polymeric micelles was 0.2 mg/mL.

In order to confirm the formation of polymeric micelles, after preparing a solvent in which PTC was self-assembled on a $D_2O$ solvent, 1H-NMR of a $D_2O$ solution of PTC was measured. PTC was completely shielded in $D_2O$ as a stearic group formed a hydrophobic core in the $D_2O$ solvent, and the peaks at $\delta$ 0.83 and 1.23, which are peaks caused by the alkylene group, were almost disappeared. The above results suggest that PEG formed a hydrophilic shell and the stearic group formed the hydrophobic core, resulting in formation of polymeric micelles.

## Preparation Example 3. Preparation of hydrophobic manganese oxidemicelle complex (PTC-M) and PC-M

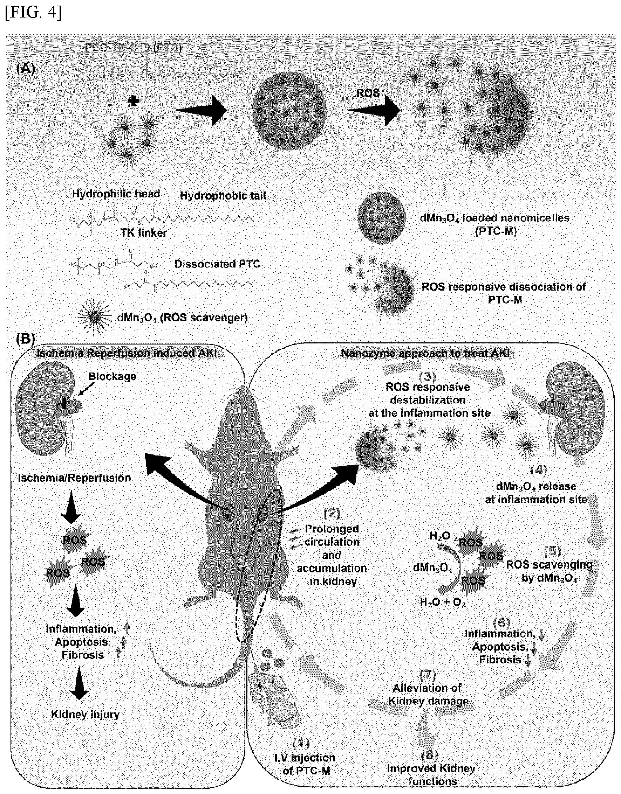

2 mg of hydrophobic manganese oxide having a C12 alkyl group of Preparation Example 1 was dissolved in 500 $\mu$L of anhydrous DMSO, 10 mg of PTC polymeric micelles were dissolved in 500 $\mu$L of anhydrous DMSO, and these two solutions were mixed to prepare a mixed solution having 1 mL total volume. After putting 20 mL of tertiary distilled water in a 50 mL tube, 1 mL of the mixed solution was added dropwise under probe sonication. At this time, the conditions of probe sonication were 30% amplitude in a pulse mode of 5 seconds on followed by 5 seconds off, and the sonication was performed for 5 minutes. The prepared solution was subjected to membrane dialysis (12-14 kDa MWCO) for one day to remove DMSO and residual impurities. Further, a qualitative filter paper (6-10 $\mu$m) was used to filter out particles with abnormal size once more. Finally, the filtered particles were lyophilized to obtain a micelle complex (PTC-M) supported with solid hydrophobic manganese oxide (FIG. 4A).

In a similar manner, ROS-insensitive nanomicelles (PC-M) loaded with hydrophobic manganese oxide nanoparticles were prepared as a control.

## Preparation Example 4. Preparation of polyethylene glycol-C18 (PC)

As a control, a ROS-insensitive polymer (PC) conjugated with stearic acid to polyethylene glycol amine (PEG-AM) was prepared. Briefly, 200 mg of m-PEG amine and 160 mg of stearic acid were mixed with 160 $\mu$l of triethylamine (TEA) in 20 ml of N,N-dimethylformamide (DMF), 480 mg of 1-ethyl-3-(3-dimethylaminopropyl)carbodiimide [EDC], and 290 mg of N-hydroxysuccinimide (NHS), followed by reacting the same at 37 °C for one day while stirring under nitrogen purging. After the reaction, the solution was purified by dialysis against distilled water for one day under the conditions of a molecular weight cut-off (MWCO) of 12-14 kDa, and then lyophilized to prepare polyethylene glycol-C18 (PC). The chemical structures of the prepared PTC and PC were investigated using proton nuclear magnetic resonance (1H NMR) spectroscopy (400 Hz, Bruker: Billerica, MA, USA) in chloroform. Empty micelles of ROS-insensitive PC were prepared by spot assembly of PC under sonication and dialysis.

**Experimental Method**

**1. Cell culture and reagents**

Briefly, for *in vitro* studies, human renal proximal tubular epithelial cells (HK-2 cells, American Type Culture Collection, Manassas, VA, USA) were used. HK-2 cells were sub-cultured in a 100 mm dish including a combination of Dulbecco's Modified Eagle's (DMEM) supplemented with 10% fetal bovine serum (FBS; Welgene), 100 U/ml penicillin and 100 mg/ml streptomycin (Sigma-Aldrich), as well as Hams F-12 medium (Welgene, Daegu, Korea) every 3 to 4 days. Then, the HK-2 cells were cultured for 24 hours at 37 °C in a humidified atmosphere of 5% $CO_2$ and 95% air and then sub-cultured until they reached 70-80% confluency. HK-2 cells were plated in a 60 mm dish in a medium containing 10% FBS and incubated for 24 hours. The cells were then cultured in DMEM-F12 medium without FBS and treated with $H_2O_2$ (1 mM) for an additional 24 hours (except 15 minutes for ERK, JNK and p38). PTC-M (2 $\mu$g) was added 1 hour before $H_2O_2$ treatment. All *in vitro* experiments were repeated twice to determine reproducibility and were performed within the 30th passage of cells.

**2. Cell viability**

*In vitro* cell viability was assessed in HK-2 cells (1 × 10$^4$ cells/well) in DMEM-F12 medium incubated overnight. The medium was aspirated and the cells were incubated at various concentrations of PTC and PTC-M for 24 hours, followed by the WST-1 assay according to the manufacturer's protocol. To compare the effects of TK ligation, the viability of cells treated with PC and PC-M was also compared. The IC50 values of the samples were analyzed in the cell viability data. Untreated cells were used as a positive control, and cells treated with 0.1% Triton x 100 were used as a negative control.

**3. Cellular uptake**

To investigate the intracellular distribution and payload release in PTC, a PTC (PTC-IR780) equipped with IR780 iodide, a near-infrared (NIR) dye, was developed. For uptake studies, HK-2 cells (3×10$^4$ cells/well) were seeded onto Lab-Tek® Chamber Slides and cultured overnight. The medium was aspirated and another sample such as IR780, PTC-IR780 or PC-IR780 containing 4 $\mu$g/ml equivalent of IR780 per well was added to the wells, followed by incubation for 4 hours. Untreated cells were used as a control. After incubation, the medium was aspirated, followed by performing DPBS wash and 4% paraformaldehyde (PFA) fixation. The cells were then counterstained with 4',6-diamidino-2-phenylindole (DAPI). A fluorescence intensity of IR780 was analyzed by fluorescence microscope (Evos FL Auto 2, Thermo Fisher Scientific) to determine IR780 uptake by cells. To confirm the effect of ROS on intracellular distribution and release, ROS conditions were mimicked by treating the cells with 200 $\mu$M $H_2O_2$ for 2 hours or culturing the cells in a hypoxia chamber for 2 days before treatment. Cellular uptake was further quantified by flow cytometry. Briefly, 1×10$^5$ HK-2 cells/well were seeded in a 12-well plate and incubated overnight. The medium was aspirated, another sample was added and incubated for 4 hours. Then, the cells were collected and centrifuged to obtain pellets, followed by two DPBS washes. The cells were then re-suspended in FACS buffer containing 3% FBS, and fluorescenceactivated cell sorting (FACS) was performed to determine the uptake capacity of each group. Data were gated with an APC-Cy 7 filter. The cellular uptake of the final PTC-M nanocomposite was determined by ICP-MS analysis. Briefly, 1×10$^5$ HK-2 cells/well were seeded in a 12-well plate and incubated overnight. The medium was then removed and different concentrations of PTC-M were added to the wells and incubated for an additional 4 hours. After incubation, the cells were collected and centrifuged to obtain pellets, which were washed twice with DPBS. Aqua regia (1 ml) was added to the final pellets for digestion, then the sample was diluted and analyzed for manganese content by ICP-MS. Untreated cells were used as a control.

**4. Flow cytometry**

Flow cytometry was performed using the Annexin V FLUOS staining kit (Sigma-Aldrich) according to the manufacturer's instructions to measure the level of Annexin V binding. Briefly, after treatment with 0 or 1 mM $H_2O_2$ for 24 hours with or without 2 $\mu$g of PTC-M pretreatment, HK-2 cells were harvested, washed twice with pre-chilled phosphate buffered saline, and re-suspended in a binding buffer containing Annexin V. After incubation in a dark room for 15 minutes, the cells were analyzed by flow cytometry (Becton-Dickinson, San Jose, CA, USA). Several controls were used to optimize instrument settings and determine gating for Windows-based platforms. Apoptotic cells were defined as PI negative and Annexin V-FITC positive.

## 5. Determination of intracellular ROS production

A fluoroprobe DCF-DA (Molecular Probes, Eugene, OR, USA) was used to determine ROS production. HK-2 cells were incubated with 0 or 1 mM $H_2O_2$ for 24 hours with or without 2 $\mu$g of PTC-M pretreatment, washed twice with Hank's balanced salt solution, and then incubated together with Hank's balanced salt solution containing DCF-DA (excluding phenol red) in a dark room at 37 °C for 30 minutes. Images were obtained with a fluorescence microscope (Nikon, Tokyo, Japan).

## 6. Experimental animals and protocols

All experimental methods were performed in accordance with relevant guidelines and regulations. The experimental protocol was approved by the Animal Care Regulations Committee of Chonnam National University Hospital (CNUHIACUC-20009).

Seven (7)-week-old male C57BL6 mice weighing 20-22 g were purchased from Samtako (Korea). Mice were housed in an animal care facility at Chonnam National University of Medicine and fed a standard diet with free access to water. Mice were divided into three groups: sham (n = 4), IRI (n = 4), and IRI with PTC-M treatment (n = 4). The surgery was performed as follows to derive the IRI mouse model. After anesthesia was induced by intraperitoneal injection of ketamine (70 mg/kg; Yuhan, Seoul, South Korea) and xylazine (7 mg/kg; Bayer, Leverkusen, Germany), a midline incision was made to expose the abdominal cavity and both nerves. To maintain body temperature, these were fixed for 30 minutes with micro clips (ROBOZ, Gaithersburg, MD, USA) on a temperature-controlled table (38.5 °C). The sham group (n = 4) went through the same procedure except that no clips were applied.

## 7. *In vivo* biodistribution

For biodistribution analysis, the IRI group was intravenously injected with PTC-IR780 at a dose of 0.5 mg/kg (IR780 equivalent). Mice were euthanized at predetermined times (6, 12, 24 and 48 hours) and major organs were collected. Fluorescence signals from the collected organs were analyzed using a fluorescencelabeled organism bio-imaging instrument (FOBI, NEO science, Gyeonggi, Korea). Fluorescence intensity of dissected major organs was measured to assess organ accumulation of PTC-IR780. Biodistribution was further compared with PC-IR780 or IR780 after administration of equivalent doses of IR780. PTC-IR780 biodistribution was also compared in normal mice after intravenous administration.

Biodistribution analysis of the final PTC-M samples was also performed. Briefly, the IRI group was injected with 0.5 mg/kg of PTC-M (Mn equivalent) sample, mice were euthanized at a predetermined time interval mentioned above, and major organs were collected. The organs were then treated with aqua regia and samples were analyzed using ICP-MS to determine tissue Mn concentrations.

To confirm *in vivo* therapeutic effects of PTC-M, a single dose of 0.5 mg/kg PTC-M was administered to the treatment group through tail vein injection after surgery. Mice were euthanized after 48 hours of reperfusion. Blood samples were collected from the heart, and the left kidney was rapidly removed and processed for western blotting or fixed in 4% paraformaldehyde solution for immunohistochemistry (IHC). Right kidneys were frozen at -80 °C for real-time polymerase chain reaction (PCR) analysis.

## 8. Plasma NGAL measurement

Plasma neutrophil gelatinase-associated lipocalin (NGAL) levels were measured using a commercial ELISA kit (R&D Systems, Minneapolis, MN, USA) according to the protocol provided by the manufacturer. A 1:400 sample dilution was performed for plasma NGAL measurement.

## 9. Semiquantitative immunoblotting

Western blot analysis was performed as follows. Kidney tissue was homogenized in an ice-cold separation solution containing 0.3 M sucrose, 25 mM imidazole, 1 mM ethylenediamine tetraacetic acid (EDTA), 8.5 mM leupeptin and 1 mM phenylmethylsulfonyl fluoride (pH 7.2). The homogenate was centrifuged at 4000 x g for 15 min at 4 °C to remove whole cells, nuclei and mitochondria. Total protein concentration was measured with a bicinchoninic acid (BCA) assay kit (Pierce; Rockford, IL, USA). All samples were adjusted to reach the same final protein concentration. Then, it was dissolved in a sample buffer containing sodium dodecyl sulfate (SDS) at 65 °C for 15 minutes and stored at -20 °C. In order to confirm equal loading of protein, initial gels were stained with Coomassie blue. SDS-polyacrylamide gel electrophoresis (PAGE) was performed on 9% or 12% polyacrylamide gels. Proteins were electrophoretically transferred onto nitrocellulose membranes (Hybond ECL RPN3032D; Amersham Pharmacia Biotech; Little Chalfont, UK) using a Bio-Rad Mini Protean II device (Bio-Rad; Hercules, CA, USA). Blots were blocked with 5% milk in PBS-T (80 mM $Na_2HPO_4$, 20 mM $NaH_2PO_4$, 100 mM NaCl and 0.1% Tween-20, pH 7.5) for 1 hour. Incubation with primary antibody overnight at 4 °C; thereafter, incubation with secondary anti-rabbit, anti-mouse or anti-goat horseradish peroxidase conjugated antibodies were performed. Immunoblots were then visualized using an enhanced chemi-luminescence system. Protein levels were quantified using a densitometry. Relative intensities of immunoblot signals were measured through densitometry using Scion Image for Windows software (Scion Corporation, 2000 - 2001. version Alpha 4.0.3.2. MD, USA) and expressed as fold-change relative to control. Primary and secondary antibodies used for immunoblotting are listed in Table 2.

[TABLE 2]

| Antibody | Host | Reactivity | Supplier | Cat. No. |
|---|---|---|---|---|
| Bax | Rabbit | Human, Mouse | Cell signaling | #2772 |
| Bcl-2 | Rabbit | Human, Mouse | Cell signaling | #3498 |
| Caspase 3 | Rabbit | Human, Mouse | Cell signaling | #9662 |
| Cleaved caspase 3 | Rabbit | Human, Mouse | Cell signaling | #9661 |
| Erk1/2 | Rabbit | Human, Mouse | Cell signaling | #9102 |
| Heme oxygenase 1 | Mouse | Mouse | Abcam | ab13248 |
| JNK | Rabbit | Human, Mouse | Cell signaling | #9252 |
| p38 | Rabbit | Human, Mouse | Cell signaling | #9212 |
| Phopho-Erk1/2 | Rabbit | Human, Mouse | Cell signaling | #9101 |
| Phopho JNK | Rabbit | Human, Mouse | Cell signaling | #9251 |
| Phospho p38 | Rabbit | Human, Mouse | Cell signaling | #9215 |
| β-actin | Rabbit | Human, Mouse | Cell Signaling | #3711 |
| Goat IgG, HRP-linked | Rabbit | Goat IgG | Sigma-Aldrich | AP106P |
| Rabbit IgG, HRP-linked | Goat | Rabbit IgG | Cell Signaling | #7074 |
| Mouse IgG, HRP-linked | Horse | Mouse IgG | Cell Signaling | #7076 |

## 10. Real-time PCR

Polymerase chain reaction analysis was performed as follows. Neocortex was homogenized in Trizol reagent (Invitrogen, Carlsbad, CA, USA). RNA was extracted with chloroform, precipitated with isopropanol, washed with 75% ethanol, and dissolved in distilled water. RNA concentration was determined by absorbance at 260 nm (Ultraspec 2000; Pharmacia Biotech, Cambridge, UK). mRNA expression of inflammatory cytokines and adhesion molecules was determined by real-time PCR. cDNA was prepared by reverse transcription of 5 μg of total RNA using oligo (dT) priming and superscript reverse transcriptase III (Invitrogen, Carlsbad, CA, USA). cDNA was quantified using the Smart Cycler II System (Cepheid, Sunnyvale, CA, USA) and SYBR Green was used for detection. Each PCR reaction was performed using 10 pM forward primer, 10 pM reverse primer, 2X SYBR Green Premix Ex Taq (TAKARA BIO INC, Seta 3-4-1, Japan), 0.5 μl cDNA and water to reach a final volume of 20 μl. Relative levels of mRNA were determined by real-time PCR using a Rotor-Gene™ 3000 Detector System (Corbette Research, Mortlake, NSW, Australia). The sequences of the primers used are shown in Table 3.

[TABLE 3]

| *Mus musculus* | Foward | Reverse |
|---|---|---|
| Ccl2 (MCP-1) | ATCCCAATGAGTAGGCTGG AGAGC (SEQ ID NO: 1) | CAGAAGTGCTTGAGGTGGT TGTG (SEQ ID NO: 2) |

(continued)

| Mus musculus | Foward | Reverse |
|---|---|---|
| Gapdh | TGTGTCCGTCGTGGATCTGA (SEQ ID NO: 3) | GATGCCTGCTTCACCACCTT (SEQ ID NO: 4) |
| Icam1 | AACTTTTCAGCTCCGGTCCTG (SEQ ID NO: 5) | TCAGTGTGAATTGGACCTGCG (SEQ ID NO: 6) |
| Il-6 | ACAACCACGGCCTTCCCTACTT (SEQ ID NO: 7) | CACGATTTCCCAGAGAACATGTG (SEQ ID NO: 8) |
| Tgfb1 (TGFβ) | CAACAATTCCTGGCGTTACCTTGG (SEQ ID NO: 9) | GAAAGCCCTGTATTCCGTCTCCTT (SEQ ID NO: 10) |
| Tnf (TNFα) | GCATGATCCGCGACGTGGAA (SEQ ID NO: 11) | AGATCCATGCCGTTGGCCAG (SEQ ID NO: 12) |
| Vcam1 | TCTCTCAGGAAATGCCACCC (SEQ ID NO: 13) | CACAGCCAATAGCAGCACAC (SEQ ID NO: 14) |

PCR was performed according to the following steps: 1) 95 °C for 5 min; 2) 95 °C for 20 seconds; 3) 58 to 60 °C for 20 seconds (optimized for each primer pair); 4) 72 °C for 30 seconds to detect SYBR Green. Further, steps 2 to 4 were repeated for an additional 40 cycles and, at the end of the last cycle, the temperature was increased from 60 °C to 95 °C in order to generate a melting curve. Reaction data were collected and analyzed with Corbett Research Software. Gene expression was calculated by performing normalization with GAPDH as an internal control using the comparison threshold obtained from the quadruplicate measurements. Melting curve analysis was performed to increase the specificity of the amplification reaction.

**11. Histology**

Kidney tissues were fixed in 4% paraformaldehyde, embedded in paraffin, and cut into 3 μm thick sections. Periodic acid-Schiff staining was performed according to the manufacturer's instructions (Abcam, Cambridge, MA, USA). For immunohistochemistry, deparaffinized tissue sections were heated at 100 °C for 15 minutes in citrate buffer with pH 6.0 (Sigma-Aldrich) to recover antigen. The sections were incubated overnight at 4 °C with primary antibody diluted in a blocking buffer (1% [w/v] bovine serum albumin dissolved in 0.3% [v/v] Triton X-100 prepared in PBS [0.3% PBS-T]). The sections were simply washed 3 times with 0.3% PBS-T and then incubated for 1 hour at room temperature with secondary antibodies diluted in blocking buffer. Vascular damage scores were calculated as follows. Vascular injury scores were measured by examination (X 200 magnification) of ≥ 10 HPF of the corticomedullary junction at the PAS-stained site (n = 5-7). The primary and secondary antibodies used for immunohistochemical analysis are listed in Table 4.

[TABLE 4]

| Antibody | Host | Reactivity | Supplier | Cat. No. |
|---|---|---|---|---|
| F4/80 | Rat | Mouse | Bio-rad | MCA497GA |
| HO-1 | Mouse | Mouse | Abcam | ab13248 |
| Rabbit IgG, HRP-linked | Goat | Rabbit IgG | Vector | PI-1000 |

(continued)

| Antibody | Host | Reactivity | Supplier | Cat. No. |
|---|---|---|---|---|
| Rat IgG, HRP-linked | Goat | Rat IgG | Vector | PI-9400 |
| Mouse IgG, HRP-linked | Goat | Mouse IgG | Vector | PI-2000 |

Apoptosis of tubular epithelial cells was detected by TUNEL staining using the ApopTag Plus Peroxidase In Situ Apoptosis Kit (Sigma-Aldrich) according to the manufacturer's instructions. Tissue sections from all major organs were collected, embedded in paraffin, and stained with hematoxylin/eosin (H&E) to compare histological changes.

## 12. Statistical analysis

Results are expressed as mean $\pm$ standard error of the mean (SEM). Multiple comparisons between three groups were performed using one-way analysis of variance (ANOVA) followed by post-hoc Tukey's honestly significant difference test. Differences with a p-value of less than 0.05 were considered significant.

## Experiment result

## 1. Analysis of hydrophobic manganese oxide and polymer composites

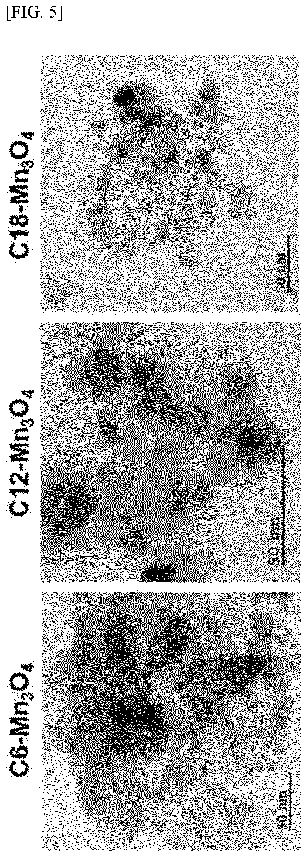

TEM image analysis of the hydrophobic manganese oxide particles prepared by the method of Preparation Example 1 was performed. As shown in FIG. 5 (scale bar 50 nm), it can be confirmed that hydrophobic manganese oxide nanoparticles are stably formed regardless of the length of an alkyl group.

For hydrophobic manganese oxide particles ($dMn_3O_4$ NPs) having a C12 alkyl group prepared by the method of Preparation Example 1, XPS spectrum analysis, EDS (Energy dispersive X-ray spectroscopy) elemental analysis, FE-TEM image analysis and XRD analysis were performed.

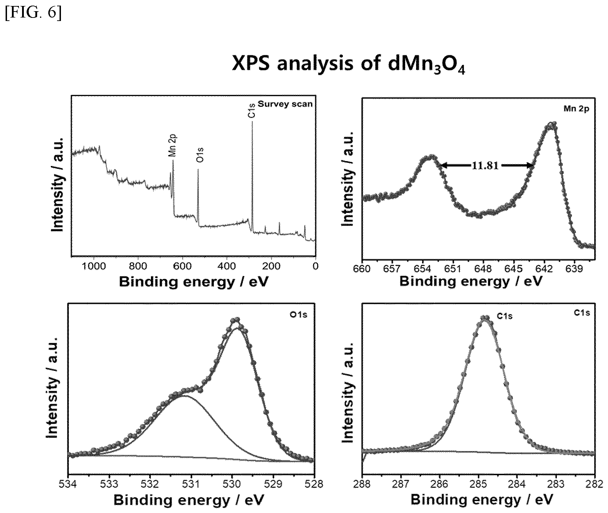

As a result of XPS spectrum analysis (FIG. 6), high peaks were confirmed in Mn 2p, O 1s and C 1s. The XPS spectrum of Mn 2P consists of two peaks located at 641.42 eV and 653.23 eV, and it was found that these peaks were attributed to Mn $2P_{3/2}$ and Mn $2P_{1/2}$, respectively. The energy gap between these two peaks is 11.81 eV, which have been matched well with the reported literature on $Mn_3O_4$.

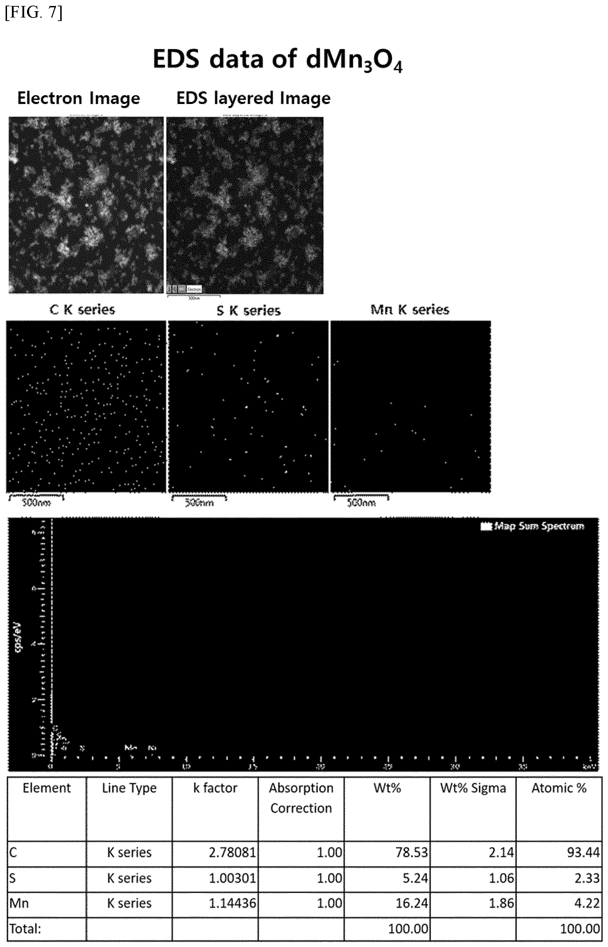

As a result of EDS elemental analysis (FIG. 7), through comparison between the position of the electron image of the hydrophobic manganese oxide nanoparticles and the position of the detected element, it was confirmed that Mn exists in $Mn_3O_4$ and elements S and C are present on SH-C12 covering the surface of the hydrophobic manganese oxide.

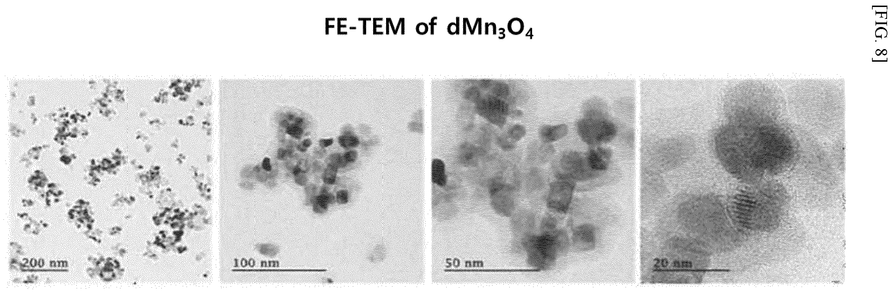

As a result of FE-TEM image analysis (FIG. 8; scale bars 200 nm, 100 nm, 50 nm and 20 nm), it was observed that the actual size of the hydrophobic manganese oxide nanoparticles was less than 20 nm ($18.0 \pm 0.6$ nm).

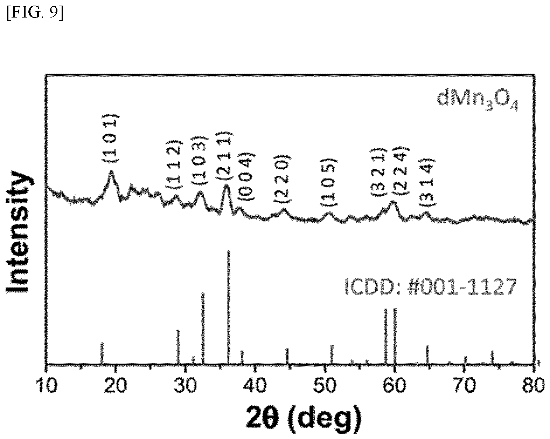

As a result of XRD analysis (FIG. 9), all diffraction peaks measured in the XRD (X-ray powder diffraction) pattern of $dMn_3O_4$ nanoparticles have been matched well with the standard pattern of hausmannite $Mn_3O_4$ [ICDD: #001-1127], therefore, high crystallinity of the nanoparticles was confirmed.

The X-ray diffraction pattern of the $dMn_3O_4$ nanoparticles shows a sharp strong peak in the XRD pattern, indicating that the synthesized sample is inherently highly crystalline. All observed peaks are well indexed to the tetragonal crystal structure. At 2θ angles of 18.0°, 28.9°, 32.5°, 36.1°, 38.1° 44.6°, 50.9°, 58.7°, 60.0° and 64.6° corresponding to (hkl) plane, diffraction peaks (101), (112), (103), (211), (004), (220), (105), (321), (224) and (314) show tetragonal spinel structures, while ($Mn_3O_4$) Hausmannite space group is 141/amd. It was found through X-ray diffraction analysis that this had the previously reported crystal structure of manganese oxide.

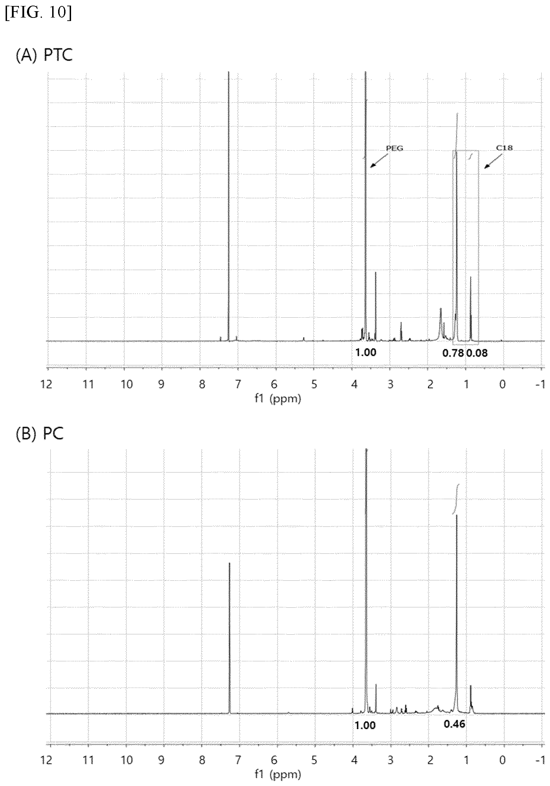

The composition of PTC and PC was analyzed by $^1$H nuclear magnetic resonance (NMR) spectroscopy (FIG. 10). The NMR spectrum showed characteristic peaks at δ 0.83 and 1.23 ppm corresponding to methyl and methylene protons of C18. The peak at δ 3.6 corresponds to $O-CH_2-CH_2-$ of PEG. The degree of substitution (DS %) of C18 in PTC and PC was calculated to be 78% and 46%, respectively.

## 2. Characteristics of PTC-M nanocomposites

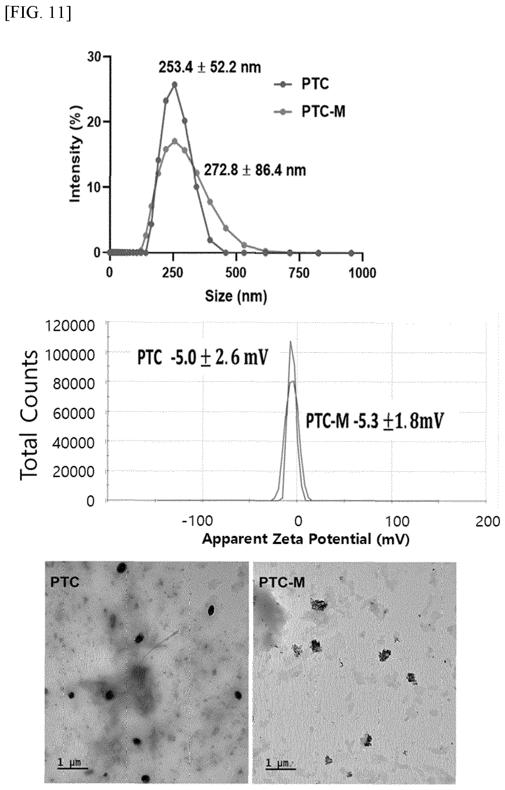

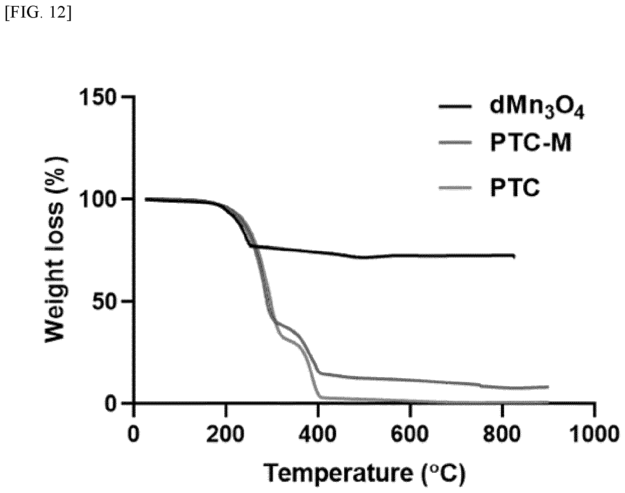

PTC-M was generated by loading $dMn_3O_4$ into ROS-sensitive nanomicelles (PTC). The $dMn_3O_4$ supply ratio (20%) of PTC was used for the preparation of PTC-M. Hydrodynamic diameters ($253.4 \pm 52.2$ nm and $272.8 \pm 86.4$ nm), zeta potentials ($-5.0 \pm 2.6$ mV and $-5.3 \pm 1.8$ mV) and FE-TEM images (scale bar 1 μm) of the prepared PTC and PTC-M are shown in FIG. 11. FE-TEM images of PTC showed spherical nanomicelles with uniform particle diameter. The hydrodynamic diameter of PTC-M was increased slightly after loading $dMn_3O_4$ because the hydrophobic space was

filled with $dMn_3O_4$, however, the zeta potential remained negative in both nanomicelles due to PEG shielding. FE-TEM images of PTC and PTC-M showed uniformly distributed particles. The loading content and encapsulation efficiency were calculated using mass spectrometry and calculated to be $10 \pm 3\%$ and $17 \pm 6\%$, respectively. The presence of $dMn_3O_4$ was clearly visible in the FE-TEM image of PTC-M, and according to EDS analysis of PTC-M, it was further confirmed that the particles showed Mn in $Mn_3O_4$, sulfur and carbon in SH-C12, and carbon, nitrogen and oxygen in PEG. A thermogravimetric (TGA) analysis was performed and $dMn_3O_4$ loading was calculated to be 12% (FIG. 12).

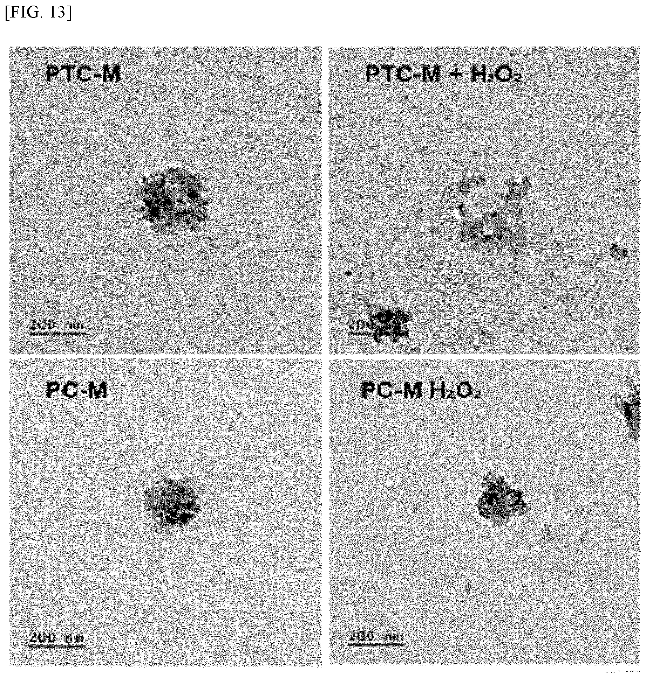

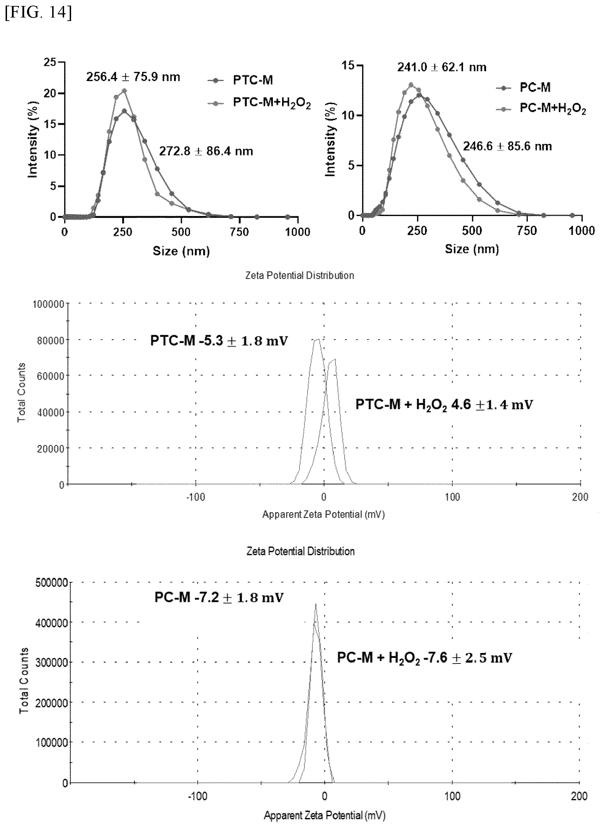

To confirm the role of TK linkage in destabilization of PTC-M's ROS response and release of $dMn_3O_4$, PTC-M was exposed to ROS conditions by adding $H_2O_2$. As a control, PC-M, which is not sensitive to ROS, was used. Both PTC-M and PC-M showed a spherical shape with nanomicelles tightly packed with $dMn_3O_4$. However, after $H_2O_2$ treatment, the upper assembly of PTC-M was distorted and $dMn_3O_4$ was released and aggregated. As shown in the FE-TEM image, PC-M showed a stable structure even after being exposed to $H_2O_2$ (FIG. 13; scale bar 200 nm). The ROS sensitivity of PTC-M was further confirmed by comparing changes in the hydrodynamic diameter and zeta potential of PC-M, as shown in Table 5 and FIG. 14. The hydrodynamic diameter and zeta potential of ROS-insensitive PC-M showed no significant difference after addition of $H_2O_2$. However, ROS-sensitive PTC-M showed a decrease in hydrodynamic size as a result of causing destabilization of the structure due to removal of the PEG shielding through TK breakage.

[TABLE5]

|  | Hydrodynamic diameter (nm) | Zeta potential (mV) |
|---|---|---|
| PTC-M | $272.8 \pm 86.4$ | $-5.3 \pm 1.8$ |
| PTC-M + $H_2O_2$ | $256.4 \pm 75.9$ | $4.6 \pm 1.4$ |
| PC-M | $246.6 \pm 85.6$ | $-7.2 \pm 1.8$ |
| PC-M + $H_2O_2$ | $241.0 \pm 62.1$ | $-7.6 \pm 2.5$ |

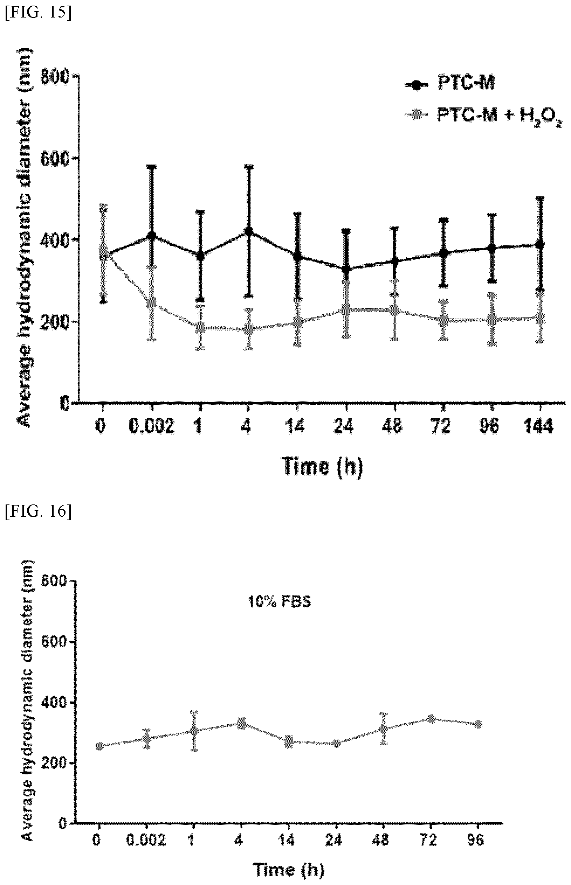

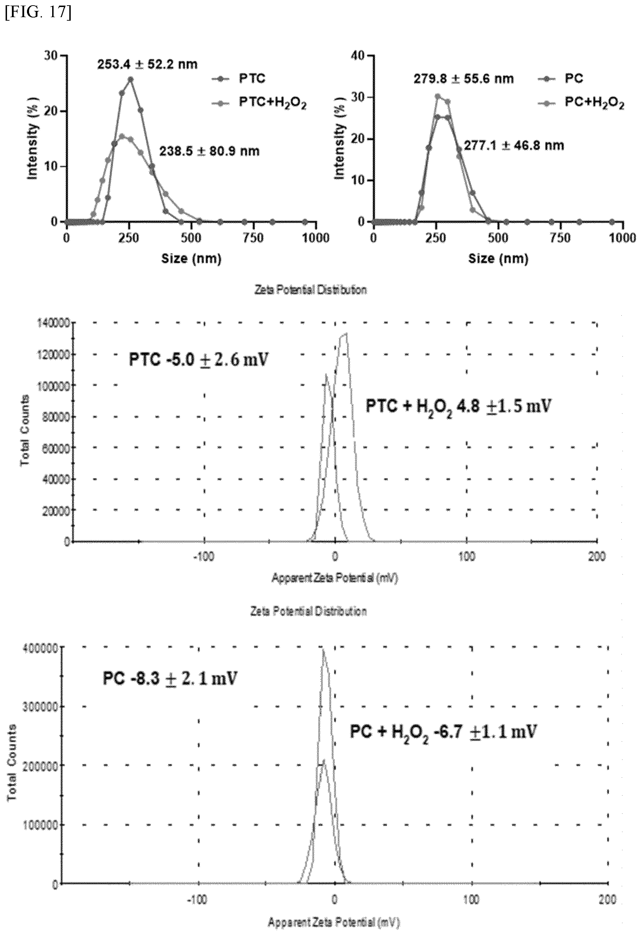

By adding $H_2O_2$, the zeta potential returned from negative charge to positive charge, which may be due to PEG removal and stearamine exposure. Size change was further confirmed by tracking the hydrodynamic diameter regardless of the presence or absence of $H_2O_2$ thereby further confirming stability over time (FIG. 15). After adding $H_2O_2$, the hydrodynamic diameter was significantly decreased within 10 seconds without further change over time up to 6 days, whereas PTC-M alone showed a stable size during the experiment, suggesting the excellent colloidal stability of PTC-M. Further, stability in 10% FBS was confirmed and it was observed that PTC-M showed good stability up to 4 days (FIG. 16). Changes in the hydrodynamic diameter and zeta potential were also confirmed in ROS-sensitive and insensitive empty nanomicelles, as shown in Table 6 and FIG. 17. These comparative studies in the presence and absence of ROS clearly demonstrated the stability and ROS sensitivity of the prepared nanomicelles or final $dMn_3O_4$ PTC-M nanozymes.

[TABLE 6]

|  | Hydrodynamic diameter (nm) | Zeta potential (mV) |
|---|---|---|
| PTC | $253.4 \pm 52.2$ | $-5.0 \pm 2.6$ |
| PTC + $H_2O_2$ | $238.5 \pm 80.9$ | $4.8 \pm 1.5$ |
| PC | $277.1 \pm 46.8$ | $-8.3 \pm 2.1$ |
| PC + $H_2O_2$ | $279.8 \pm 55.6$ | $-6.7 \pm 1.1$ |

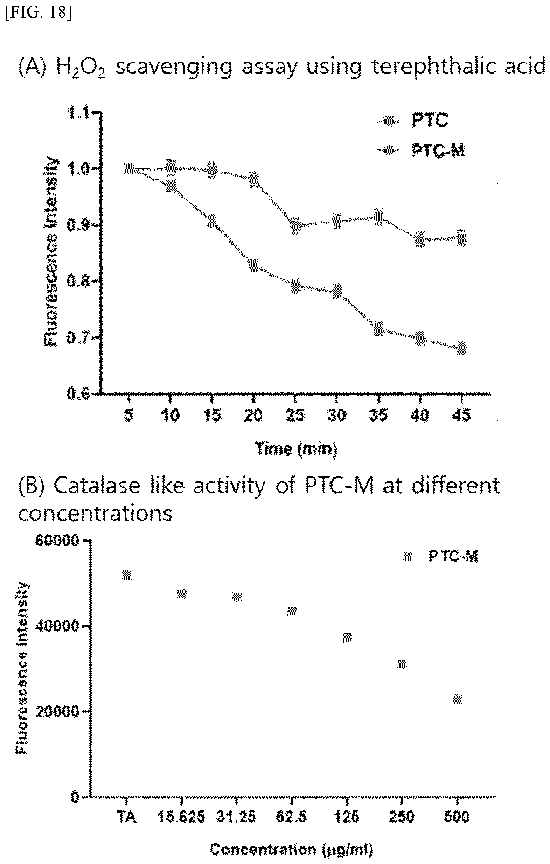

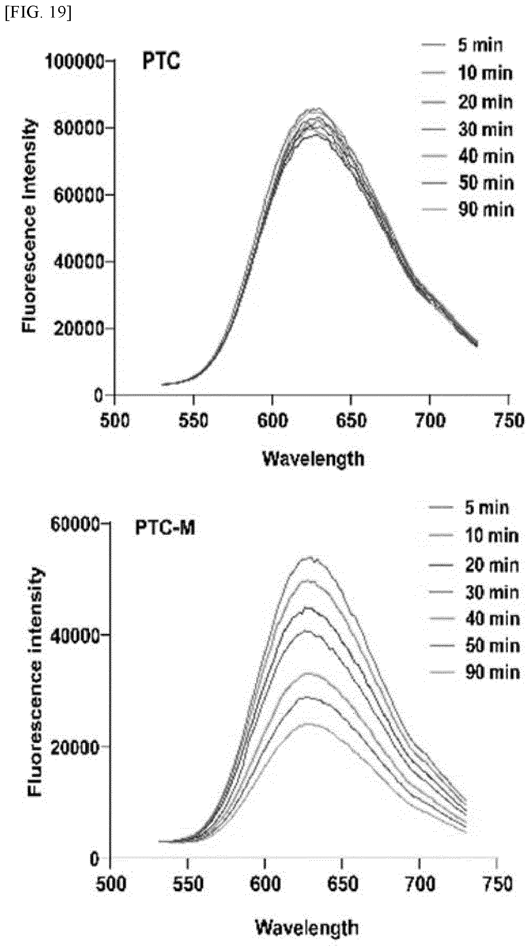

The $H_2O_2$ removal characteristics of the final PTC-M nano-composites due to $dMn_3O_4$NP loading were further confirmed. This was tested using terephthalic acid (TA) as a fluorescent probe. TA reacted with $H_2O_2$ to form 2-hydroxy terephthalic acid with a fluorescence peak at 425 nm. Since $dMn_3O_4$ converts $H_2O_2$ into water and oxygen, the fluorescence intensity is reduced, thus exhibiting catalase-like activity as shown in FIG. 18A. PTC-M showed a strong decrease in fluorescence intensity over time compared to empty micelle PTC in the presence of $H_2O_2$. The catalase-like activity of PTC-M was also concentration dependent, as shown in FIG. 18B. The ability of PTC-M to produce oxygen was further confirmed using a $O_2$-specific fluorescent probe Ru(ddp) in the presence of $H_2O_2$. The fluorescence intensity of Ru(ddp) was decreased in the presence of $O_2$, suggesting efficient production of $O_2$. As shown in FIG. 19, the fluorescence intensity of Ru(ddp) was decreased over time in the presence of $H_2O_2$ in the PTC-M group, whereas the presence of $H_2O_2$ in the PTC alone group showed very little decrease in fluorescence. From this, catalase activity causing oxygen production in PTC-M could be confirmed. Finally, $H_2O_2$ removability of PTC-M was investigated using an oxygen detector.

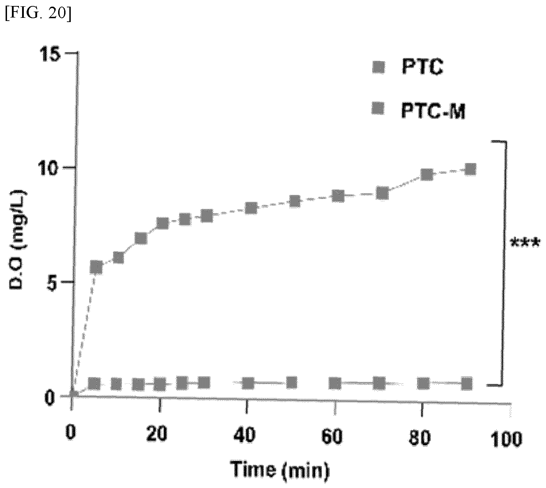

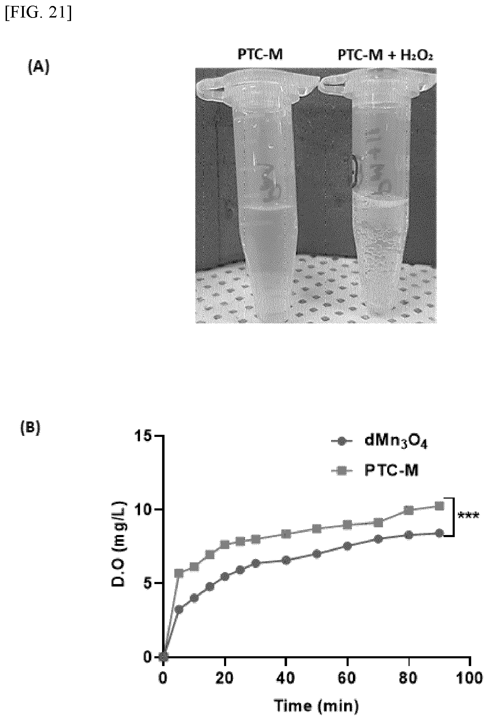

As shown in FIG. 20, oxygen production by PTC-M was increased within an initial period of time and then gradually increased, whereas empty micelle PTC did not show oxygen production. An imbalance of $H_2O_2$ was observed by the formation of oxygen bubbles under $H_2O_2$ in the PTC-M group, as shown in FIG. 21A. Further, it was also observed that PTC-M exhibited higher oxygen production than $dMn_3O_4$ alone (FIG. 21B). This may be probably due to the close packing of $dMn_3O_4$ in PTC-M, which is more useful for the reaction compared to free $dMn_3O_4$. Although the nanomicelles were loosened by $H_2O_2$ treatment, as shown in FE-TEM, $dMn_3O_4$ may still exist inside PTC-M, such that the catalase activity is higher than that of free $dMn_3O_4$. All these results confirmed together the excellent catalase mimetic activity of PTC-M.

### 3. *In vitro* cell viability and cell uptake

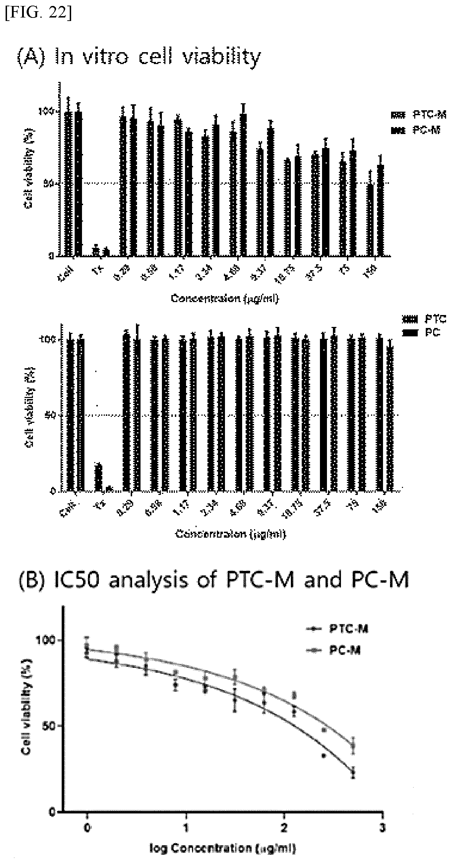

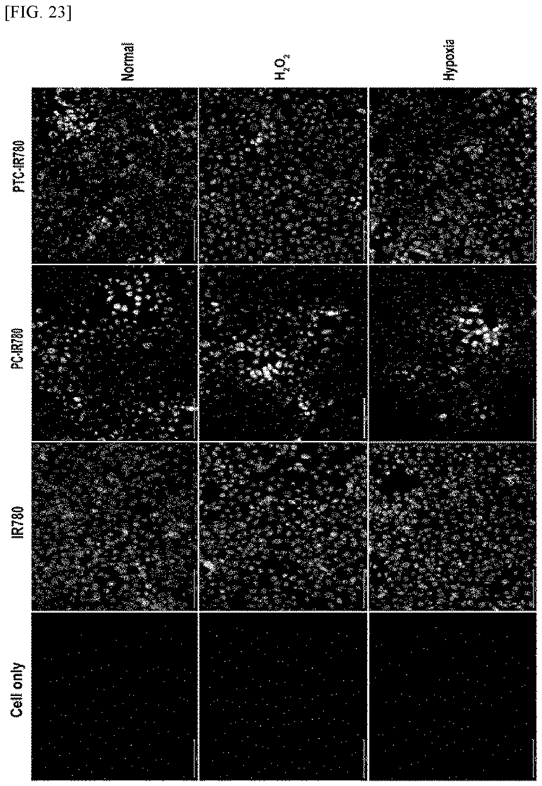

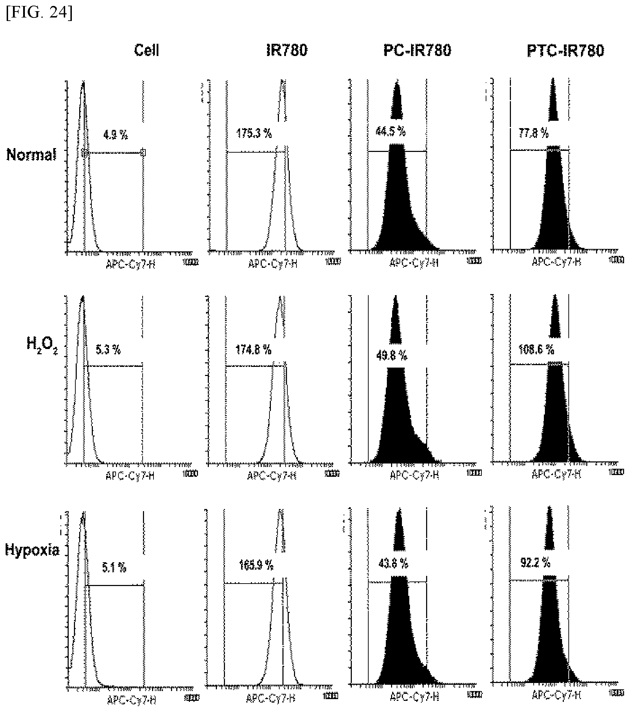

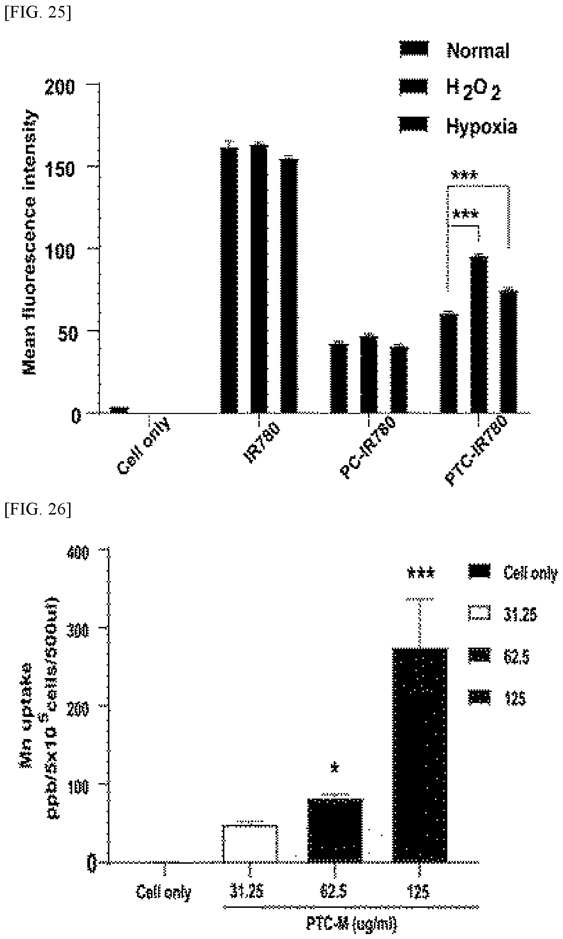

In order to investigate cell uptake, ROS sensitivity of ROS sensitive PTC, and payload release pattern of PTC, PTC was compared with PC under *in vitro* conditions. First, cell viability of the prepared PTC, PC, PTC-M and PC-M was investigated in HK-2 cells by WST-1 assay (FIG. 22A; at each concentration, left bar: PTC-M, PTC; right bar: PC-M, PC). Empty micelles showed almost no apoptosis, and cell viability was decreased after $dMn_3O_4$-loading, however, the change was not statistically significant. Since the empty micelles were not cytotoxic, the slight change in cell viability could be due to $dMn_3O_4$ adsorbed on the surface or unexpected stearamine exposure in the group. IC50 values of PTC-M and PC-M were determined to be 130.41 and 247.17 $\mu g/ml$, respectively (FIG. 22B). To further investigate the intracellular uptake and distribution of nanomicelles, IR780-loaded nanomicelles were prepared to visualize the fluorescence intensity of IR780 in PTC-IR780 using a fluorescence microscope (Evos FL Auto 2, Thermo Fisher Scientific, USA) (FIG. 23). As a control, the uptake of PC-IR780, which is not sensitive to ROS, was analyzed. Compared to PC-IR780, PTC-IR780 showed a broader and higher uptake in HK-2 cells. The exact reason for the enhanced uptake of PTC-IR780 in non-ROS conditions is unclear, but could be presumed that PTC may show some sensitivity to normal ROS levels in cell culture conditions, resulting in stripping of PEG and leaving a slightly positive surface charge. PC-IR780 showed a similar uptake pattern regardless of ROS induced by $H_2O_2$ treatment or hypoxia-induced ROS conditions, whereas PTC-IR780 had a different distribution pattern inside the cells due to enhanced release of IR780 inside the cells. As observed in the cell viability data, the increased PTC uptake due to stearamine may be responsible for the increased cell death (apoptosis) caused by PTC-M. IR780 iodide, a hydrophobic dye, showed a higher uptake rate regardless of nanomicelle loading. PTC-IR780 showed higher fluorescence intensity than PC-IR780. Cellular uptake was further analyzed by determining the mean fluorescence intensity (MFI) using fluorescence activated cell sorting (FACS) analysis that supports the data obtained by fluorescence microscope (FIG. 24). As shown in the drawing, MFI was highest in the PTC-IR780 group, which is sensitive to ROS, compared to other groups, and it was confirmed from this result that the release of IR780 from the cells was improved. MFI of various groups supporting microscopic data suggesting that PC-IR780 showed no significant difference in cellular uptake even under ROS-inducing conditions, whereas PTC-IR780 showed a significant increase in cellular uptake when treated under ROS-inducing conditions, was further quantified (FIG. 25; Normal, $H_2O_2$, Hypoxia in order from the left bar). Cellular uptake of PTC was also determined by measuring residual Mn in the cells after treatment with PTC-M. As shown in FIG. 26, PTC-M exhibited concentration dependent uptake in HK-2 cells. Therefore, these results suggest accelerated release of IR780 inside cells due to enhanced internalization of nanomicelles and ROS-mediated degradation of nanomicelles.

### 4. Effect of PTC-M nanocomposite on inflammation and apoptosis of HK-2 cells stimulated with hydrogen peroxide

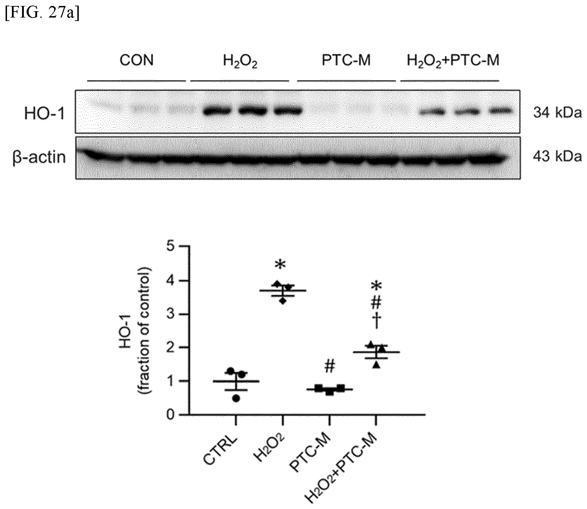

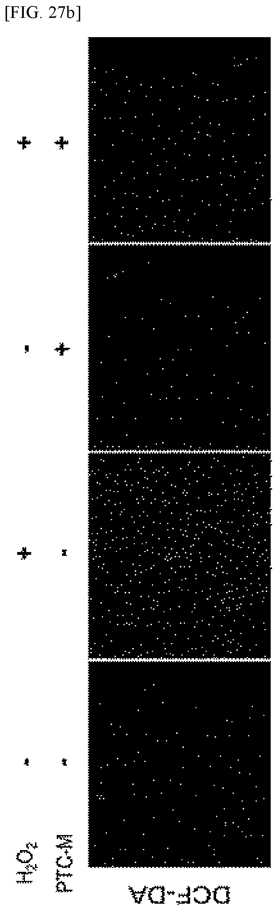

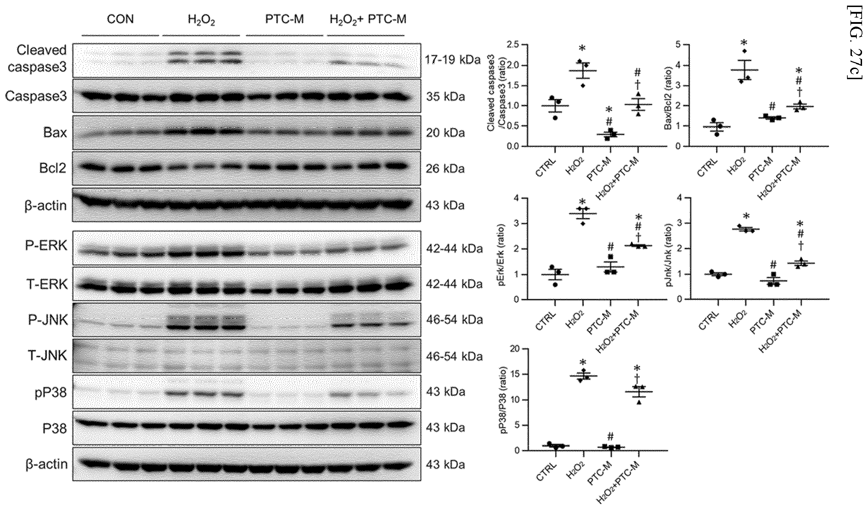

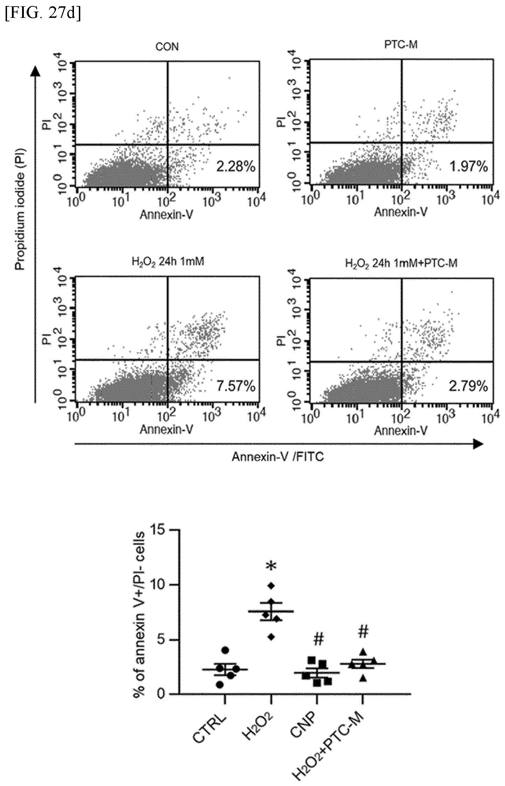

In order to investigate the effect of PTC-M on oxidative stress conditions at the cellular level, *in vitro* studies were performed in HK-2 cells. HK-2 cells were stimulated with $H_2O_2$, a representative ROS. $H_2O_2$ enhanced heme-oxygenase (HO)-1 expression, which was improved by co-treatment with PTC-M (FIG. 27A). Intracellular ROS changes were confirmed by 2',7'-dichlorodihydrofluorescein diacetate (DCF-DA) assay. HK-2 cells stimulated with $H_2O_2$ showed high fluorescence intensity, but the fluorescence intensity was decreased after co-treatment with PTC-M (FIG. 27B). The cleaved caspase3/Caspase3 ratio, pErk/Erk ratio, pP38/P38 ratio, BAX/BCL-2 ratio, and pJnk/Jnk ratio were increased by $H_2O_2$ treatment, which were also restored by simultaneous treatment with PTC-M, thus exhibiting protection against to apoptosis (FIG. 27C). HK-2 cells treated with $H_2O_2$ showed a significant progressive increase in Annexin $V^+/PI^-$ staining, which was prevented by PTC-M (FIG. 27D). ERK, JNK, and p38 phosphorylation induced by $H_2O_2$ were also inhibited by co-treatment with PTC-M, but no significant decrease in phosphorylated P38 was observed (FIG. 27C). These data suggest that PTC-M inhibits the apoptotic process induced by ROS in renal tubular epithelial cells.

### 5. Biodistribution of PTC-M nanocomposites in IRI mice

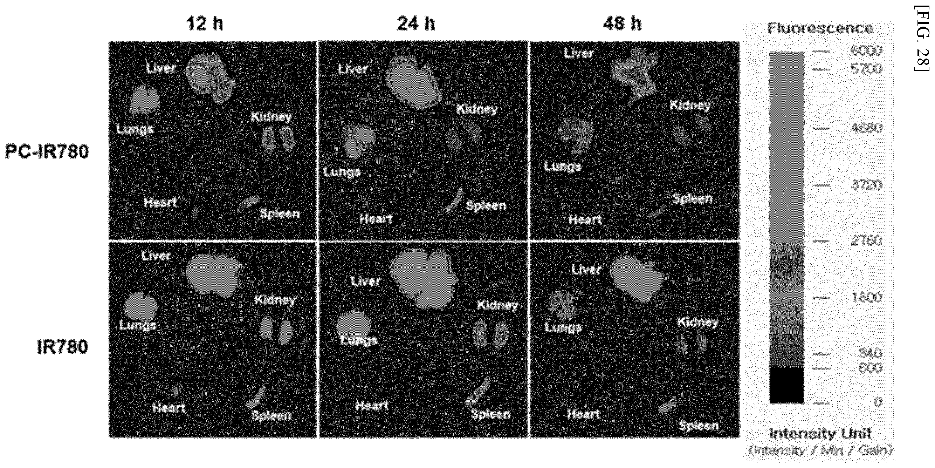

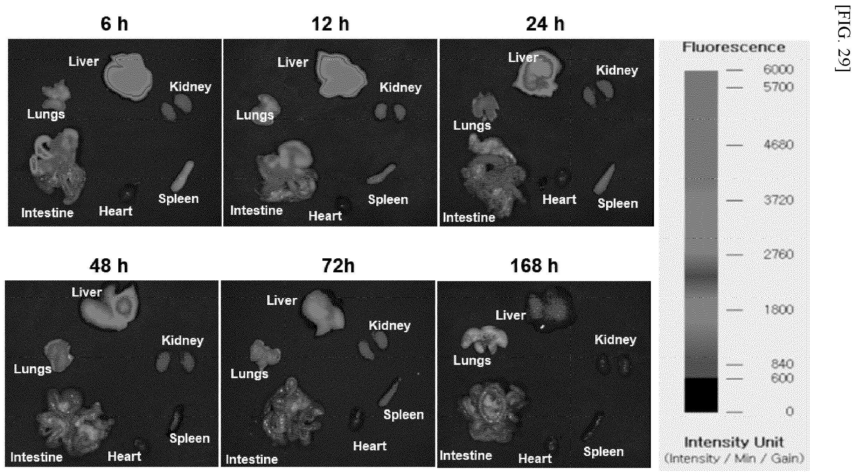

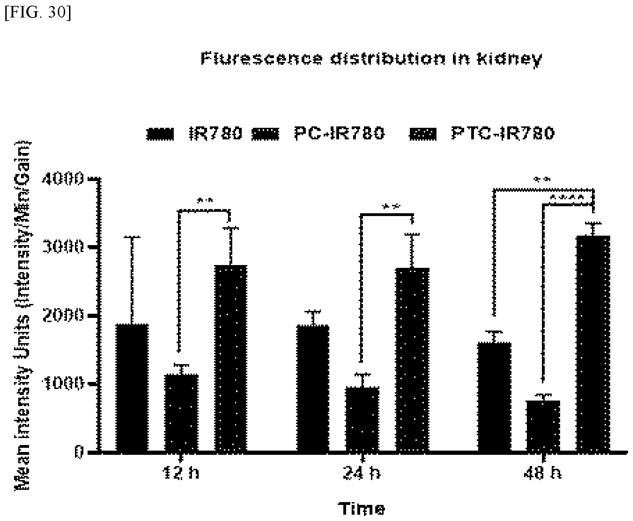

The biodistribution of PTC in IRI mice was investigated by loading the fluorescent dye IR780 as a model drug (PTC-IR780). The biodistribution of PC-IR780 and IR780 alone was compared, and all groups showed fluorescence in major organs (liver, lung, heart, spleen and kidney), but PTC-IR780 exhibited enhanced accumulation in the kidney, unlike the PC-IR780 or IR780 groups (FIG. 28). Further, the biodistribution profile of PTC-IR780 in normal mice was investigated and it was found that nanoparticles showed general higher liver accumulation after intravenous injection, but not much accumulation in kidney (FIG. 29; liver, lung, intestine, heart, spleen and kidney). Therefore, it could be concluded that the enhanced renal accumulation of PTC was mainly mediated by renal inflammation. In this example, PTC nanomicelles are designed in such a way that these are gradually accumulated in the inflammatory site while circulating in the blood. After reaching the inflammatory site, PTC was destabilized by the presence of enhanced ROS, and the loaded IR780 remaining in the kidney was released for a longer time to enhance fluorescence. As a result of comparing kidney accumulation among all groups by quantifying *ex vivo* fluorescence intensity, it was observed that PTC showed fluorescence in other major organs inevitable in the case of systemic injection, but showed predominant kidney accumulation (FIG. 30; IR780, PC-IR780, PTC-IR780 in order from the left bar).

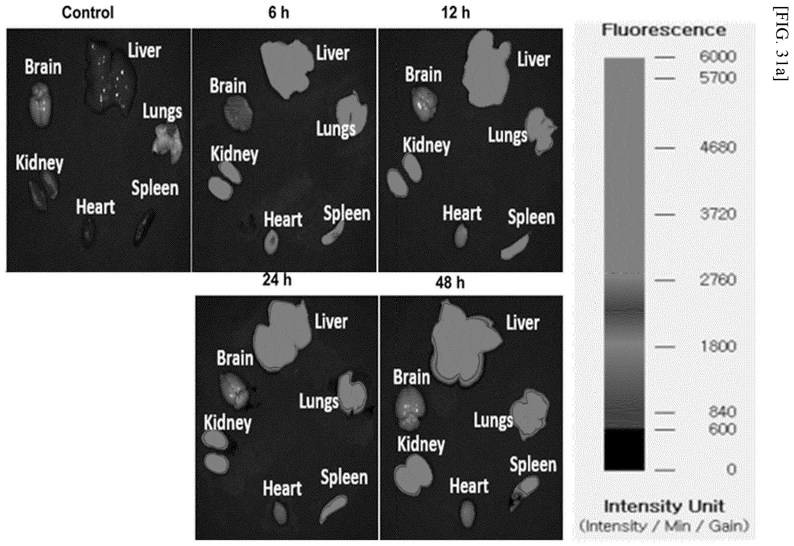

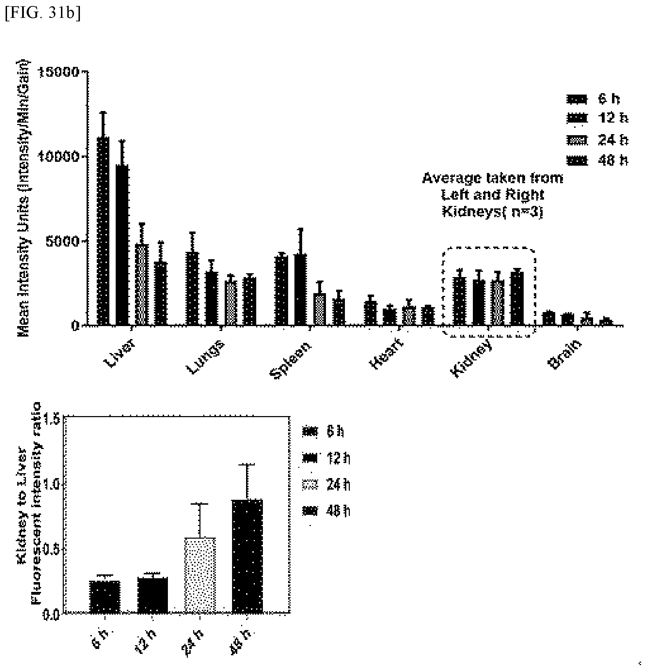

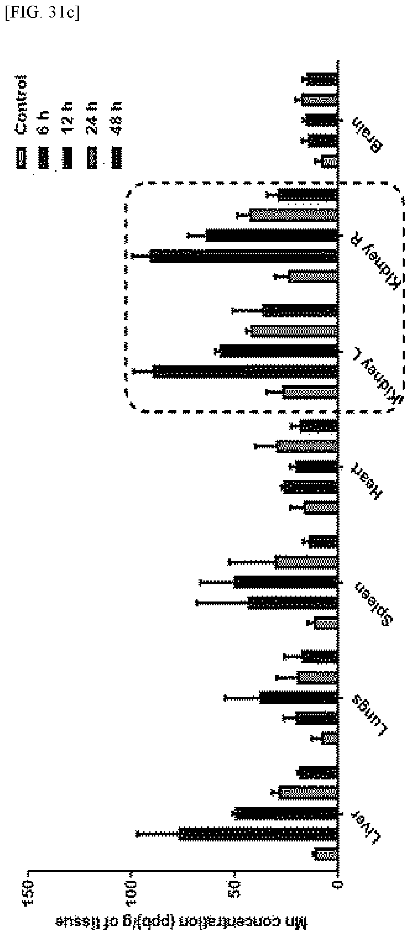

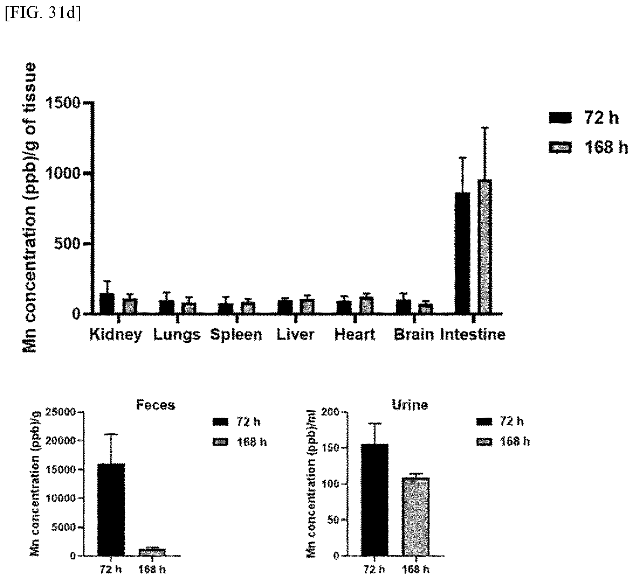

As shown in FIG. 31A, strong IR780 fluorescence intensity was observed in the kidney from 6 hours after PTC-IR780 injection. Fluorescence intensities of major organs (liver, lung, spleen, heart, kidney and brain) were found to be high in order of liver, lung, spleen and kidney. Fluorescence intensities in other organs were decreased over time, whereas fluorescence intensities in kidneys remained relatively constant up to 48 hours. Therefore, the fluorescence intensity of liver vs kidney tended to increase gradually over time (FIG. 31B). The biodistribution was further confirmed by determining the Mn content in other organs through inductively coupled plasma mass spectrometry (ICP-MS) analysis. As shown in FIG. 31C, the Mn concentration in both kidneys peaked at 6 hours after injection, and was gradually decreased until 48 hours. When tissue Mn concentrations were compared with other major organs, at some point the kidneys reached higher tissue Mn concentrations than the liver, lungs and spleen. To confirm the clearance of PCT-M, ICP-MS analysis was performed on all organs, feces and urine at the end of the study and one week later, and it was observed that most of the Mn appeared in the intestine and feces (FIG. 31D). It has been observed that Mn clearance predominantly takes a hepatobiliary route rather than renal clearance.

## 6. PTC-M nanocomposites reduce kidney injury markers and attenuate morphological changes in IRI mouse kidneys.

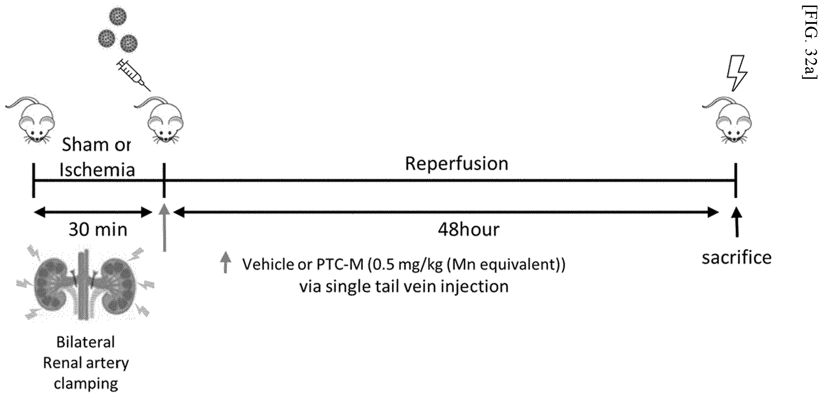

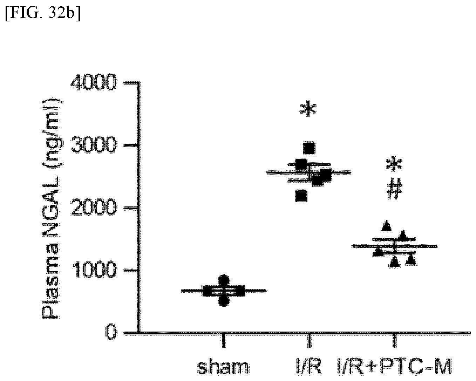

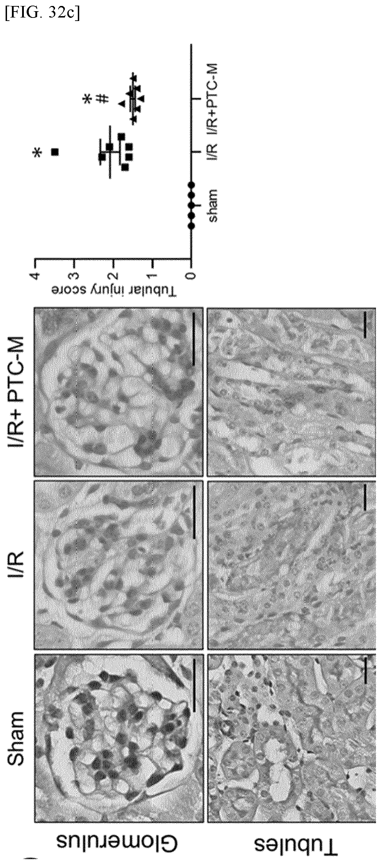

An *in vivo* experiment was performed to determine the efficacy of PTC-M in reducing renal damage in renal IRI (FIG. 32A). Plasma neutrophil gelatinase-associated lipocalin (NGAL), a well-known marker of tubular injury in AKI, was measured to investigate the overall damage status of the IRI kidney. The level of plasma NGAL was significantly higher in the IRI group than in the sham group, but was reduced by PTC-M treatment (FIG. 32B). Periodic acid-Schiff (PAS) staining was performed on kidney sections to confirm morphological changes (FIG. 32C). In contrast to sham mice, renal tubular edema and swelling filled with necrotic material were observed. Inflammatory cells infiltrating the interstitial space were observed in the kidneys of IRI mice. These changes were ameliorated by PTC-M treatment. In IRI mice treated with PTC-M, tubular edema, tubular necrotic material and inflammatory cell infiltration were relatively attenuated. Unlike changes in tubules, no significant morphological difference was observed in glomeruli in each group.

## 7. PTC-M ameliorates inflammation and apoptosis in IRI mouse kidney.

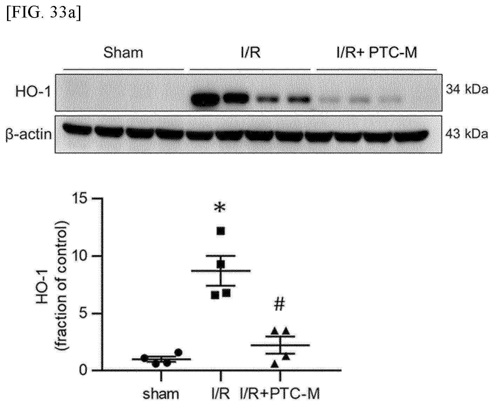

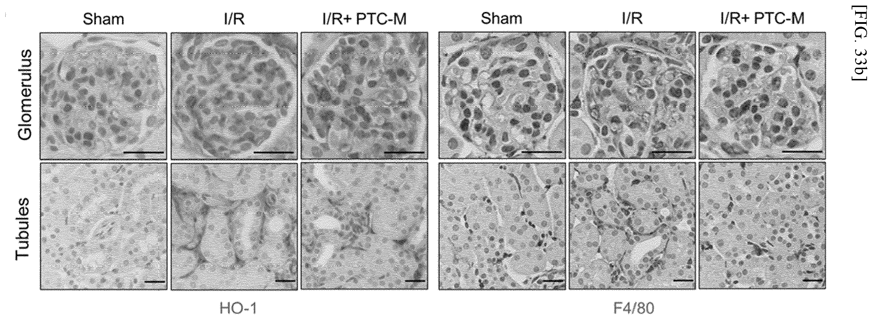

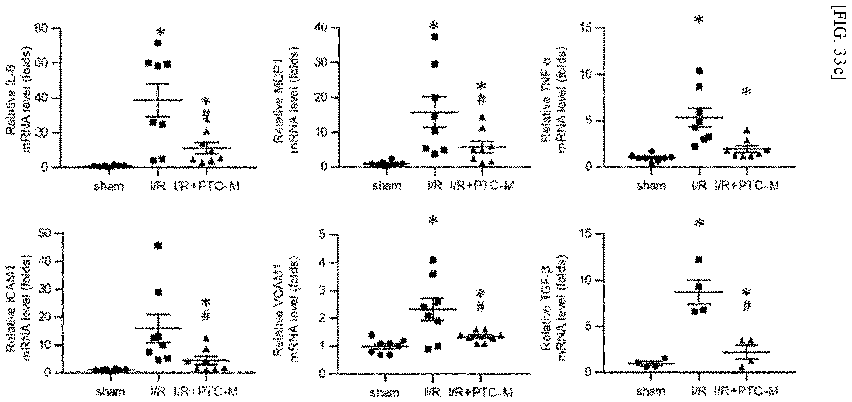

To evaluate the degree of oxidative stress in the IRI mouse kidney, the expression level of HO-1 was investigated through immunoblot analysis. It was found an increase in expression levels of HO-1 in the kidneys of IRI mice attenuated by PTC-M treatment (FIG. 33A). Immunohistochemical staining for HO-1 showed increased expression in glomerular mesangium, periglomerular space and peritubular interstitium of IRI mouse (I/R) kidneys, which was significantly reduced in PTC-M treated mouse kidneys. (FIG. 33B). Evidence of active inflammation in the kidneys of IRI mice demonstrated, by quantitative polymerase chain reaction (qPCR) analysis, that transcript levels of pro-inflammatory cytokines and adhesion molecules were higher (FIG. 33C). For interleukin (IL)-6, monocyte chemo-attractant protein (MCP)-1, tumor necrosis factor (TNF)-$\alpha$, intracellular adhesion molecule (ICAM)-1, vascular cell adhesion molecule (VCAM-1), and transforming growth factor ((TGF)-$\beta$), mRNA expression levels were significantly increased in the kidneys of IRI mice, and such increase was suppressed by administration of PTC-M (FIG. 33C). Immunohistochemical staining for F4/80, a marker of murine macrophages, showed increased expression in the periglomerular and interstitial spaces of the kidneys of IRI mice, which was also attenuated by PTC-M treatment (FIG. 33B). Taken together, these data suggest that PTC-M inhibits renal inflammation and oxidative stress in IRI mice.

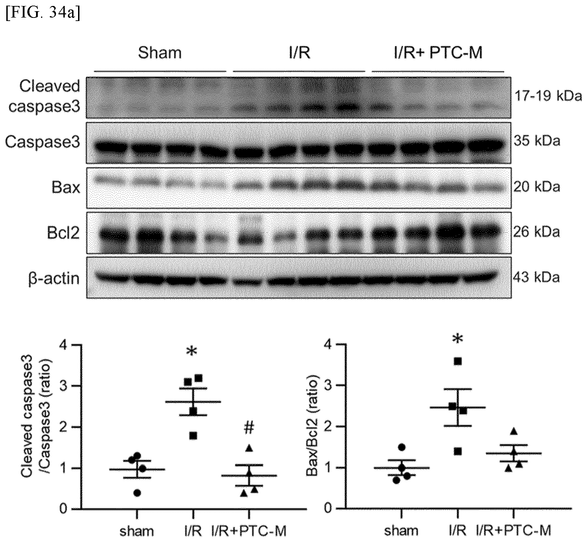

Variation in apoptotic proteins was evaluated to evaluate the degree of tubular cell death in IRI mice. Immunoblots showed increased Bax/Bcl-2 ratio and cleaved caspase 3/caspase 3 ratio in kidney sections of IRI mice, suggesting that apoptosis was aggravated (FIG. 34A). Such variation was not observed in mice treated with PTC-M. PTC-M treatment clearly reduced the apoptosis process of renal tubular cells induced by oxidative stress.

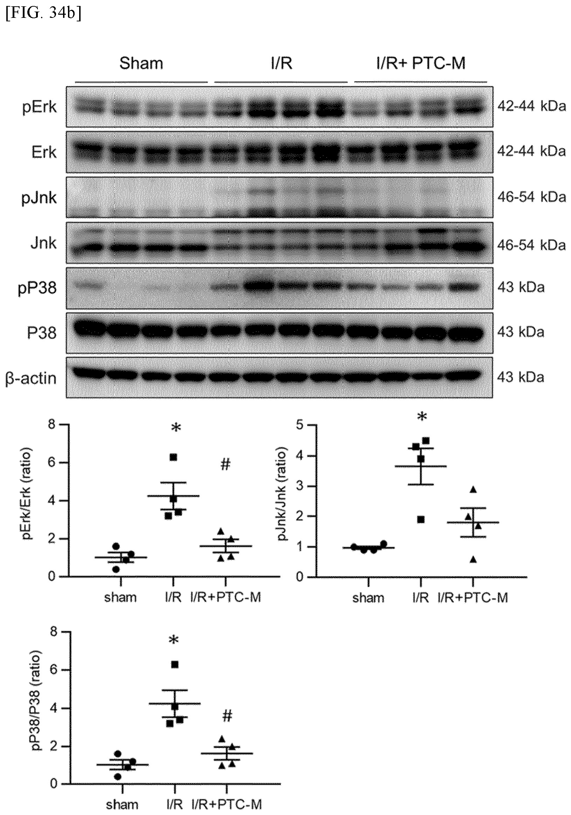

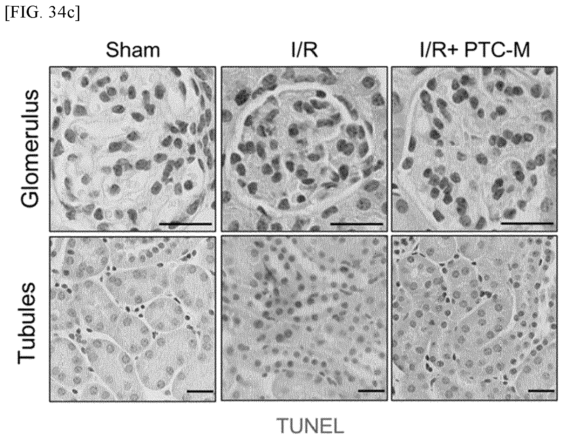

To confirm whether PTC-M inhibits ROS-mediated apoptosis through mitogenactivated protein kinase (MAPK), changes in the MAPK signaling pathway were analyzed. Immunoblot analysis of MAPK pathway members and phosphorylated (activated) forms was performed (FIG. 34B). Levels of phosphorylated ERK, JNK and P38 were enhanced in the kidneys of IRI mice compared to sham mice attenuated by PTC-M, but no significant decrease in phosphorylated JNK was observed. Terminal deoxynucleotidyl transferase dUDP nickend labeling (TUNEL) staining showed more TUNEL-positive tubular epithelial cells in the renal cortex of IRI mice compared to sham mice, but PCT-M treatment reduced the number of TUNEL-positive cells (FIG. 34C).

These results suggest that PTC-M reduced apoptosis in the kidneys of IRI mice by affecting the MAPK signaling pathway. These findings in the IRI mouse kidney are consistent with results in $H_2O_2$-stimulated human kidney cells, revealing a general mechanism of PTC-M renal protection.

## Claims

1. A pharmaceutical composition for treating inflammatory diseases, comprising: manganese oxide particles having hydrophobic substituents on the surface thereof; and micelles that support the particles therein and are formed of micelle-forming molecules in which a hydrophilic portion and a hydrophobic portion are bound by a reactive oxygen species (ROS) cleavable linker.

2. The pharmaceutical composition according to claim 1, wherein the hydrophobic substituent is a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group or a halogen-containing group.

3. The pharmaceutical composition according to claim 1, wherein the manganese oxide is any one of MnO, $Mn_2O_3$, $MnO_2$, $Mn_3O_4$ and $Mn_2O_5$.

4. The pharmaceutical composition according to claim 1, wherein the manganese oxide particles have a particle diameter of 10 to 30 nm.

5. The pharmaceutical composition according to claim 1, wherein the linker is a thioketal linker (thioketal), TSPBA linker ($N^1$-(4-boronobenzyl)-$N^3$-(4-boronophenyl)-$N^1$,$N^1$,$N^3$,$N^3$-tetramethylpropane-1,3-diaminium) or AA linker (aminoacrylate).

6. The pharmaceutical composition according to claim 1, wherein the hydrophilic portion includes a water-soluble polymer, the hydrophobic portion includes a hydrophobic hydrocarbon group, and the reactive oxygen species (ROS) cleavable linker is a thioketal linker.

7. The pharmaceutical composition according to claim 6, wherein the water-soluble polymer is polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol.

8. The pharmaceutical composition according to claim 6, wherein the hydrophobic hydrocarbon group is a hydrocarbon group derived from hexadecylamine, heptadecylamine, stearamine, nonadecylamine, dodecylamine, N,Nbis(2-hydroxyethyl)laurylamine, linoleic acid, linolenic acid, palmitic acid, oleylamine, hexadecanoic acid, stearic acid, oleic acid, eicosenoic acid or erucic acid.

9. The pharmaceutical composition according to claim 1, wherein the manganese oxide particles are included in an amount of 5 to 10 parts by weight based on 100 parts by weight of the micelles.

10. The pharmaceutical composition according to claim 1, wherein the micelles have a particle diameter of 150 to 250 nm.

11. The pharmaceutical composition according to claim 1, wherein the inflammatory disease is renal failure, atopic dermatitis, edema, dermatitis, allergy, asthma, conjunctivitis, periodontitis, rhinitis, otitis media, sore throat, tonsillitis, pneumonia, gastric ulcer, gastritis, Crohn's disease, colitis, hemorrhoids, gout, ankylosing spondylitis, rheumatic fever lupus, fibromyalgia, psoriatic arthritis, osteoarthritis, rheumatoid arthritis, periarthritis, tendonitis, tenosynovitis, myositis, hepatitis, cystitis, nephritis, sjogren's syndrome or multiple sclerosis.

12. The pharmaceutical composition according to claim 1, further comprising a bioactive substance supported inside the micelles.

[FIG. 1]

-> A schematic diagram of hydrophobic $Mn_3O_4$ nanoparticle

[FIG. 2]

Step 1:

HS───COOH + Acetone ──HCl (g)──→ Thiol Ketal Linker (TL)

3-mercaptopropionic acid

Step 2:

PEG-AM + TL ──EDC/NHS──→ PEG-TL

Step 3:

PEG-TL + Ocetadecylamine

──EDC/NHS──→

Hydrophilic    ROS sensitive    Hydrophobic

PTS

[FIG. 3]

EP 4 410 274 A1

[FIG. 4]

[FIG. 5]

[FIG. 6]

# XPS analysis of dMn₃O₄

[FIG. 7]

# EDS data of dMn₃O₄

**Electron Image**     **EDS layered Image**

**C K series**     **S K series**     **Mn K series**

| Element | Line Type | k factor | Absorption Correction | Wt% | Wt% Sigma | Atomic % |
|---|---|---|---|---|---|---|
| C | K series | 2.78081 | 1.00 | 78.53 | 2.14 | 93.44 |
| S | K series | 1.00301 | 1.00 | 5.24 | 1.06 | 2.33 |
| Mn | K series | 1.14436 | 1.00 | 16.24 | 1.86 | 4.22 |
| Total: | | | | 100.00 | | 100.00 |

[FIG. 8]

FE-TEM of dMn$_3$O$_4$

[FIG. 9]

[FIG. 10]

(A) PTC

(B) PC

[FIG. 11]

[FIG. 12]

[FIG. 13]

[FIG. 14]

[FIG. 15]

[FIG. 16]

[FIG. 17]

[FIG. 18]

## (A) H$_2$O$_2$ scavenging assay using terephthalic acid

## (B) Catalase like activity of PTC-M at different concentrations

[FIG. 19]

[FIG. 20]

[FIG. 21]

(A)

PTC-M          PTC-M + H₂O₂

(B)

[FIG. 22]

(A) In vitro cell viability

(B) IC50 analysis of PTC-M and PC-M

[FIG. 23]

[FIG. 24]

[FIG. 25]

[FIG. 26]

[FIG. 27a]

[FIG. 27b]

| H₂O₂ | - | + | - | + |
|---|---|---|---|---|
| PTC-M | - | - | + | + |

DCF-DA

[FIG. 27c]

[FIG. 27d]

[FIG. 28]

[FIG. 29]

[FIG. 30]

Flurescence distribution in kidney

[FIG. 31a]

[FIG. 31b]

[FIG. 31c]

[FIG. 31d]

[FIG. 32a]

EP 4 410 274 A1

Sham or Ischemia

Reperfusion

30 min

48hour

Vehicle or PTC-M (0.5 mg/kg (Mn equivalent)) via single tail vein injection

sacrifice

Bilateral Renal artery clamping

[FIG. 32b]

[FIG. 32c]

[FIG. 33a]

[FIG. 33b]

[FIG. 33c]

[FIG. 34a]

[FIG. 34b]

[FIG. 34c]

## INTERNATIONAL SEARCH REPORT

| International application No. |
|---|
| **PCT/KR2022/009070** |

### A. CLASSIFICATION OF SUBJECT MATTER

**A61K 9/107**(2006.01)i; **A61K 47/69**(2017.01)i; **A61K 47/20**(2006.01)i; **A61K 47/16**(2006.01)i; **A61K 33/32**(2006.01)i; **A61P 29/00**(2006.01)i

According to International Patent Classification (IPC) or to both national classification and IPC

### B. FIELDS SEARCHED

Minimum documentation searched (classification system followed by classification symbols)

A61K 9/107(2006.01); A23L 33/10(2016.01); A61K 47/69(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 소수성(hydrophobic), 산화망간(manganese oxide), 담지(loading), 양친매성 (amphipathic), 활성산소-분해성 링커(ROS-cleavable linker), 마이셀(micelle), 염증(inflammation)

### C. DOCUMENTS CONSIDERED TO BE RELEVANT

| Category* | Citation of document, with indication, where appropriate, of the relevant passages | Relevant to claim No. |
|---|---|---|
| A | HOWELL, M. et al. Manganese-loaded lipid-micellar theranostics for simultaneous drug and gene delivery to lungs. Journal of Controlled Release. 2013, vol. 167, pp. 210-218.<br>See abstract; page 213, right column; and figure 1. | 1-12 |
| A | UTHAMAN, Saji et al. Tumor microenvironment-responsive nanoparticles for cancer theragnostic applications. Biomaterials Research. 2018, vol. 22, no. 3, pp. 172-182.<br>See page 179. | 1-12 |
| A | JI, Hao et al. Recent advances in ROS-sensitive nano-formulations for atherosclerosis applications. Pharmaceutics. 11 September 2021, vol. 13, 1452, pp. 1-17.<br>See pages 3, 6, 8 and 12. | 1-12 |
| A | SUN, Changzhen et al. A ROS-responsive polymeric micelle with a π-conjugated thioketal moiety for enhanced drug loading and efficient drug delivery. Organic & Biomolecular Chemistry. 2017, vol. 15, pp. 9176-9185.<br>See entire document. | 1-12 |

✓ Further documents are listed in the continuation of Box C.    ✓ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**28 December 2022** | Date of mailing of the international search report<br><br>**30 December 2022** |
| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2019)

## INTERNATIONAL SEARCH REPORT

| International application No. |
|---|
| **PCT/KR2022/009070** |

**C.    DOCUMENTS CONSIDERED TO BE RELEVANT**

| Category* | Citation of document, with indication, where appropriate, of the relevant passages | Relevant to claim No. |
|---|---|---|
| A | HOU, Xiaoya et al. Novel dual ROS-sensitive and CD44 receptor targeting nanomicelles based on oligomeric hyaluronic acid for the efficient therapy of atherosclerosis. Carbohydrate Polymers. 2020, vol. 232, 115787, pp. 1-8. See entire document. | 1-12 |
| A | KR 10-2012-0068450 A (THE CATHOLIC UNIVERSITY OF KOREA INDUSTRY-ACADEMIC COOPERATION FOUNDATION) 27 June 2012 (2012-06-27) See entire document. | 1-12 |
| PX | KR 10-2022-0008243 A (DR. CURE) 20 January 2022 (2022-01-20) See paragraphs [0068]-[0072]; and claims 1-11. | 1-10,12 |

Form PCT/ISA/210 (second sheet) (July 2019)

**INTERNATIONAL SEARCH REPORT**

| International application No. |
|---|
| **PCT/KR2022/009070** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

| | | International application No. |
|---|---|---|
| | | **PCT/KR2022/009070** |

| Patent document cited in search report | Publication date (day/month/year) | Patent family member(s) | Publication date (day/month/year) |
|---|---|---|---|
| KR 10-2012-0068450 A | 27 June 2012 | KR 10-1705374 B1 | 09 February 2017 |
| KR 10-2022-0008243 A | 20 January 2022 | KR 10-2395946 B1 | 10 May 2022 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Remington's Pharmaceutical Sciences. 1995